# EUROPEAN PATENT SPECIFICATION

(11) **EP 2 322 118 B1**
(45) Date of publication and mention of the grant of the patent: **19.12.2012**
(21) Application number: 09014130.0
(22) Date of filing: 11.11.2009
(51) Int. Cl.: A61F 2/06, A61M 29/00, A61F 2/84

(54) **Medical devices for medical device delivery systems**
Medizinische Vorrichtungen für Systeme zur Bereitstellung einer medizinischen Vorrichtung
Dispositifs médicaux pour systèmes de fourniture de dispositif médical

(43) Date of publication of application: 18.05.2011
(73) Proprietor: Abbott Laboratories Vascular Enterprises Limited, Dublin 2 (IE)
(72) Inventor: Grandt, Axel, 72479 Strassberg (DE)
(74) Representative: Peters, Hajo

(56) References cited:
- EP-A1- 1 637 177
- WO-A1-01/05462
- WO-A1-2008/024621
- WO-A1-2009/066330
- US-A1- 2003 212 450
- US-A1- 2004 073 155
- US-A1- 2005 203 606
- US-A1- 2008 208 118

## Description

The present disclosure relates to medical treatment devices. In particular, the present disclosure relates to systems and methods for interluminal medical treatment devices.

### BACKGROUND OF THE INVENTION

Stents, grafts, endoprostheses and a variety of other implantable medical devices are used in interventional procedures, such as for lining or repairing vessel walls, for filtering fluid flow, and for expanding or scaffolding occluded or collapsed vessels. Such endoprostheses can be delivered and used in an accessible body lumen of a human or animal, and can be deployed by any of a variety of recognized means.

One recognized use of implantable medical devices, such as stents, is for the treatment of atherosclerotic stenosis in blood vessels. For example, after a patient undergoes a percutaneous transluminal coronary angioplasty or similar interventional procedure, an endoprosthesis, such as a stent, can be deployed at the treatment site to improve the results of the medical procedure and to reduce the likelihood of restenosis.

However, current implantable medical devices may be limited with respect to the quantity, variation, and duration of beneficial agent treatments that the implantable medical devices can deliver to an interluminal treatment site. In addition, current endoprostheses may be unable to deliver beneficial agent in a precise, controlled, and accurate manner. Furthermore, current methods of interluminal medical treatment may be unable to isolate and protect beneficial agents from fluid flow within a lumen that can carry the beneficial agents away from the targeted treatment site.

Therefore, it is an object of the present invention to overcome the above-mentioned limitations and to provide a medical device suitable to isolate a targeted treatment site from fluid flow within a lumen. In addition, it is an object of the present invention to provide a method for treating a targeted treatment site within a lumen, whereby beneficial agent is delivered at the targeted site without being carried away by fluid flow.

A delivery catheter is disclosed in US 2008/0208118. For delimiting claim 1 over the prior art, document WO 2008/024621 is used.

### SUMMARY OF THE INVENTION

The object of the present invention is solved by embodiments according to claim 1. For example, the present invention concerns an interluminal medical treatment device configured to treat a targeted treatment site within a lumen. In particular, the device can include an elongated generally tubular outer member having an expandable proximal portion, an expandable distal portion, and a central portion having a lesser expandability than the proximal portion and distal portion. The device can also include at least one membrane coupled to the outer member. The outer member and at least one membrane may at least partially define a housing area extending around the central portion of the outer member and positioned longitudinally between the proximal portion and distal portion of the outer member. The device may also include an elongated generally tubular inner member disposed within the outer member and at least one membrane. The inner member can include at least one lumen.

In one preferred embodiment according to claim 1 the object of the present invention is solved by an intraluminal medical treatment device configured to treat a targeted treatment site within a lumen, comprising: an elongated generally tubular outer member comprising an expandable proximal portion, an expandable distal portion, and a central portion, wherein the central portion has a lesser expandability than the proximal portion and the distal portion and at least one membrane coupled to the outer member, wherein the outer member and the at least one membrane at least partially define a housing area extending around the central portion of the outer member and positioned longitudinally between the expanded proximal portion and the expanded distal portion of the outer member.

A method for treating a targeted treatment site within a lumen comprises the steps of deploying a medical device according to the present invention within the lumen proximate the targeted treatment site, and introducing at least one beneficial agent into the housing area.

It is to be understood that the foregoing general description and the following detailed description are exemplary and explanatory only and are not to be viewed as being restrictive of the present disclosure, as claimed. Further advantages of this disclosure will be apparent after a review of the following detailed description of the disclosed embodiments, which are illustrated schematically in the accompanying drawings and in the appended claims.

### BRIEF DESCRIPTION OF THE DRAWINGS

In order to describe the manner in which the above-recited and other advantages and features of the present disclosure can be obtained, a more particular description of the implementations briefly described above will be rendered by reference to specific configurations thereof which are illustrated in the appended drawings. Understanding that these drawings depict only typical configurations of the present disclosure and are not therefore to be considered to be limiting of its scope, the implementations of the present disclosure will be described and explained with additional specificity and detail through the use of the accompanying drawings.

FIG. 1A illustrates a perspective view of an example interluminal medical treatment device in accordance with an implementation of the present disclosure;

FIG. 1B illustrates a side view of the example interluminal medical treatment device of FIG. 1A;

FIG. 1C. illustrates a cut-away view of the example interluminal medical treatment device of FIG. 1B along the line 1C-1C;

FIG. 2A illustrates a perspective view of an additional example interluminal medical treatment device in accordance with an implementation of the present disclosure;

FIG. 2B illustrates a side view of the example interluminal medical treatment device of FIG. 2A;

FIG. 2C illustrates a cut-away view of the example interluminal medical treatment device of FIG. 2B along the line 2C-2C;

FIG. 3A illustrates a perspective view of a further example interluminal medical treatment device in accordance with an implementation of the present disclosure;

FIG. 3B illustrates a side view of the example interluminal medical treatment device of FIG. 3A;

FIG. 3C illustrates a cut-away view of the example interluminal medical treatment device of FIG. 3B along the line 3C-3C;

FIG. 4A illustrates a perspective view of a still further example interluminal medical treatment device in accordance with an implementation of the present disclosure;

FIG. 4B illustrates a side view of the example interluminal medical treatment device of FIG. 4A;

FIG. 4C illustrates a cut-away view of the example interluminal medical treatment device of FIG. 4B along the line 4C-4C;

FIG. 4D illustrates a cut-away view of the example interluminal medical treatment device of FIG. 4B along the line 4D-4D;

FIG. 5 illustrates an exemplary subject for an interluminal medical treatment device; and

FIGS. 6A-6F illustrate various steps in the deployment of an example interluminal medical treatment device.

It should be noted that the figures are not drawn to scale and that elements of similar structures or functions are generally represented by like reference numerals for illustrative purposes throughout the figures. It also should be noted that the figures are only intended to facilitate the description of example configurations of the present disclosure.

### DETAILED DESCRIPTION

The present disclosure relates to an interluminal medical treatment device, delivery system, and methods of interluminal medical treatment.

As outlined above, there is a need in the prior art to provide medical devices suitable to isolate a targeted treatment site from fluid flow within a lumen. Such isolation would allow delivering an agent to a targeted treatment site and in parallel to protect the agent from external influence, such as fluid flow within the lumen.

Therefore, a first example is an intraluminal medical treatment device configured to treat a targeted treatment site within a lumen, comprising: an elongated generally tubular outer member comprising an expandable proximal portion, an expandable distal portion, and a central portion, wherein the central portion has a lesser expandability than the proximal portion and the distal portion and at least one membrane coupled to the outer member, wherein the outer member and the at least one membrane at least partially define a housing area extending around the central portion of the outer member and positioned longitudinally between the expanded proximal portion and the expanded distal portion of the outer member.

Embodiments of the invention allow an agent to be delivered to a targeted treatment site while protecting the agent from external influence, such as fluid flow. When the interluminal medical device according to present invention is deployed within a lumen proximate a targeted treatment site, the interluminal medical treatment device can define, and direct lumen fluid flow away from, a housing area. A drug or other beneficial agent can thus be introduced into the housing area and protected from fluid flow so as to be efficiently delivered to the targeted treatment site.

The tubular outer member of the interluminal medical treatment device according to the present invention comprises a proximal portion and a distal portion separated by a central portion. The proximal portion and the distal portion expand during deployment to engage with a lumen. The central portion, which is connected or integrally formed between the proximal portion and the distal portion, has less expandability. Therefore, the central portion may have a smaller expanded outside diameter than the expanded outside diameters of the expandable proximal portion and expandable distal portion. As a result, a housing area is formed between at least the central portion and the vessel. The proximal portion and the distal portion essentially seal or close the housing area as they engage with the walls of the vessel.

The at least one membrane of the interluminal medical treatment device according to the present invention is attached to the central portion and/or at least a portion of the distal portion and the proximal portion of the outer member. Thereby, the at least one membrane is either disposed within and coupled to an inner surface of the outer member or outside and coupled to an outer surface of the outer member. Such a configuration redirects fluid flow within the body lumen away from the housing area and through an inner passageway defined by the inside surface of the membrane and/or outer member. Thus, the housing area is at least partially isolated from fluid flow within the body lumen. Accordingly, the at least one membrane prevents fluid in the lumen from entering the housing area. Therefore, beneficial agent(s) within the housing area may be at least partially protected from being carried away from the targeted treatment site by fluid flow within the body lumen resulting in a protected and improved treatment of a targeted treatment site within a body lumen.

In one embodiment the at least one membrane of the interluminal medical treatment device according to the present invention has some porosity and/or permeability. Preferably, the at least one membrane is substantially permeable, semi-permeable, impermeable, substantially porous, or non-porous. Thereby, the permeability and/or porosity may vary longitudinally of the at least one membrane. In a preferred embodiment of interluminal medical treatment device according to the present invention the at least one membrane is permeable to gas and/or oxygen. Such permeability has the advantage that oxygen from blood can still reach the walls of the vessels within the housing area. Therefore, it is possible to let the interluminal medical treatment device according to the present invention remain at the site of treatment while ischemia is prevented.

In one further embodiment of the interluminal medical treatment device the at least one membrane is comprising or is consisting of material selected from the group comprising polymers, polyurethanes, synthetic fabrics, elastomers, polyamides, silicone, PTFE, and/or ePTFE.

In one preferred embodiment of the interluminal medical treatment device according to the present invention the at least one membrane comprises a proximal membrane disposed at least partially within the proximal portion of the outer member and a distal membrane disposed at least partially within the distal portion of the outer member. Preferably, the membrane may each be umbrella-shaped extending approximately half way into the proximal and distal portions, respectively. As described herein, however, the membrane may have some porosity. More preferably, the proximal membrane has a different permeability or porosity than the distal membrane; most preferably with the distal membrane being less permeable than the proximal membrane.

In another preferred embodiment of the interluminal medical treatment device according to the present invention the membrane extends along the proximal portion, the central portion and distal portion of the outer member.

In one embodiment the outer member of the interluminal medical treatment device according to the present invention is formed by a plurality of struts. Thereby, the number and configuration of the struts may vary as desired. In a preferred embodiment the struts of the outer member of the present invention are formed of shape memory material. Preferably, the shape memory material is selected from the group comprising nickel titanium and/or alloys thereof, copper-aluminum-nickel and/or alloys thereof, copper chromium and/or alloys thereof, stainless steel, other metals, polymers, biodegradable materials, bioresorbable materials, bioabsorbable materials, other similar materials, and/or combinations thereof. More preferably, one or more beneficial agents or radio-opaque markers are incorporated into the material of and/or coated over at least a portion of the outer member.

In one embodiment the interluminal medical treatment device according to the present invention further comprises an elongated generally tubular inner member. Preferably, the inner member is disposed within the outer member and the at least one membrane. More preferably, the inner member extends - at least partly - longitudinally along the length of the interluminal medical treatment device.

The interluminal medical device may further include additional lumens that are longitudinally placed inside of the device. These lumens are used for various purposes such as for a guide wire during deployment and/or placement of the device, to assist in fluid flow through the device, and for delivery of a beneficial agent to the housing area.

Embodiments of the invention contemplate the delivery of a beneficial agent to the housing area. The beneficial agent is thus constrained within the housing area and can be targeted to a specific site without interference from fluid flow in the lumen.

In one embodiment of the interluminal medical treatment device according to the present invention the inner member comprises at least one lumen selected from the group comprising a perfusion lumen, a beneficial agent lumen, and/or a guidewire lumen. In one preferred embodiment the beneficial agent lumen and/or the guidewire lumen are disposed within the perfusion lumen. Preferably, the beneficial agent lumen extends from a point external to a patient receiving treatment through the lumen and into the housing area. As a result, a physician may use a syringe or other external device to introduce a beneficial agent into the housing area through the beneficial agent lumen. Alternatively, the beneficial agent lumen is coupled to an automated device, whether internal or external to the patient, configured to automatically inject a predetermined amount of beneficial agent into the housing area at predetermined time intervals. Once a beneficial agent was introduced into the housing area it may - if deemed necessary - also be removed again from the housing area using the beneficial agent lumen. Generally thus, material comprised or accumulating in the housing area may be removed from the housing area by use of the beneficial agent lumen, including debris like broken cells or pieces of plaques accumulating during treatment of the targeted treatment site with the intraluminal medical device according to the invention.

In a further embodiment of the interluminal medical treatment device according to the present invention the inner member further comprises one or more corresponding perfusion port(s) and/or beneficial agent port(s). The perfusion port(s) and the beneficial agent port(s) are in fluid communication with the perfusion lumen and benefical agent lumen, respectively. Preferably, the beneficial agent port(s) extend into the housing area, or the beneficial agent lumen of the interluminal medical treatment device is in fluid communication with the housing area by way of the beneficial agent port(s). In addition, the inner member may further comprise one or more perfusion port(s) proximal of the housing area to allow fluid flow within the body lumen that has been redirected by the membrane and/or outer member to enter into and travel through the perfusion lumen of the inner member. Preferably, the inner member also comprises perfusion port(s) (also defined as perfusion exists) distal of the housing area to allow fluid flow to exit the perfusion lumen. As a result, the housing area can be isolated and protected since fluid flow within the body lumen is redirected through the inner member.

In a further embodiment of the interluminal medical treatment device according to the present invention a guidewire lumen extends longitudinally the inner member comprising also perfusion ports and a perfusion lumen being in fluid communication. In one preferred embodiment the guidewire lumen is disposed within the perfusion lumen and is joined with the perfusion lumen. Joining is to be understood as being in fluid communication. Accordingly, the joined fuidewire lumen is through two or more openings in fluid communication with the perfusion lumen, thus in principle increasing the overall volume for perfusion through the inner member. Preferably, a corresponding guidewire passes through the guidewire lumen and is used to position the interluminal medical treatment device proximate a targeted treatment site within the body lumen. Once the interluminal medical treatment device is positioned and/or deployed, the guidewire may be removed from, remain within the guidewire lumen or retreated through the guidewire lumen in proximal direction until at least the part of the guidewire lumen which is joined to the perfusion lumen is free from the guidewire. Thus, in the embodiments, where the guide wire lumen is disposed within the perfusion lumen, removing the guidewire results in a greater perfusion lumen. Thus, retrieving the guidewire proximal of the perfusion port(s) results in a greater size (or diameter) of the perfusion member between the perfusion port(s) and the perfusion exit(s) than the diameter of the remaining inner member. Therefore, in a preferred embodiment the size of the perfusion lumen varies or may be varied longitudinally.

In a further embodiment of the interluminal medical treatment device according to the present invention the at least one lumen comprised in the inner member is comprising a perfusion lumen and the inner member includes at least one perfusion port in a proximal portion of the inner member and at least on perfusion port in a distal portion of the inner member, the perfusion ports being in fluid communication with the perfusion lumen, and wherein the diameter of the perfusion lumen between at least one perfusion port in a proximal portion of the inner member and at least one perfusion port in a distal portion of the inner member is greater than the diameter of the remaining inner member not comprising a perfusion lumen.

In one embodiment the interluminal medical treatment device according to the present invention is disposed within an elongated generally tubular delivery device in a collapsed position. The delivery device according the present invention comprises a distal portion and a proximal portion and is suitable to deliver the interluminal medical treatment device near a targeted treatment site within a body lumen. In addition, the interluminal medical treatment device according to the present invention may be retrieved and/or deployed using such a delivery device. In a preferred embodiment of the interluminal medical treatment device according to the present invention delivery device is a catheter.

In another embodiment the interluminal medical treatment device according to the present invention further comprises a retrieval member coupled to the proximal portion of the outer member. The retrieval member is suitable to be used to deploy the interluminal medical treatment device. In particular, pushing the retrieval member in a distal direction can force the interluminal medical treatment device out of the delivery device. Upon leaving the delivery device, the outer member and membrane may unfold and/or expand into a deployed position, thereby engaging the inner wall of the body lumen and defining a housing area proximate the targeted treatment site. Beneficial agent(s) may then be introduced into the housing area to treat the targeted treatment site. In addition, the interluminal medical treatment device may be removed from the body lumen by pulling the retrieval member in a proximal direction relative to the delivery device. Thus, the interluminal medical treatment device may be collapsed and pulled into the delivery device. Thereafter, the interluminal medical treatment device may be completely retrieved from the body. In a preferred embodiment of the interluminal medical treatment device according to the present invention the retrieval member comprises a plurality of elongated struts. Preferably, the elongated struts of the retrieval member extend at least partially into the delivery device.

In one embodiment the interluminal medical treatment device according to the present invention further comprises at least one injection device. In one preferred embodiment of the interluminal medical treatment device according to the present invention the at least one injection device is disposed through the delivery device and/or extends at least partially into the housing area of the expanded outer member. Preferably, the injection device extends from a point external to a patient receiving treatment through the lumen and into the housing area. As a result, a physician may use a syringe or other external device to introduce a beneficial agent into the housing area through the injection device. Alternatively, the injection device may be coupled to an automated device, whether internal or external to the patient, configured to automatically introduce a predetermined amount of beneficial agent into the housing area at predetermined time intervals. In a further preferred embodiment, the injection device is temporarily positioned within the housing area and then removed once a beneficial agent is introduced into the housing area. Alternatively, the injection device may remain within the housing area for an extended period of time, thereby facilitating sustained and/or ongoing treatments of a targeted treatment site within the body lumen. The injection device may be connected to the outer member, membrane and/or retrieval member. In additional embodiments, the interluminal medical treatment device may include multiple injection devices extending into the housing area, through which the same or different beneficial agents may be introduced. Furthermore, one or more beneficial agents may be introduced through a single injection device. In the embodiments of the present invention the size of the injection device may vary as necessary for a particular configuration. In one example embodiment, the injection device may include a 27 gauge, or larger, needle. The injection device may also be configured for the delivery of cells to the housing area. Preferably, the injection device of the interluminal medical treatment device according to the present invention is an injection catheter or a needle. Once a beneficial agent was introduced into the housing area it may - if deemed necessary - also be removed again from the housing area using the injection device/s. Generally thus, material comprised or accumulating in the housing area may be removed from the housing area by use of the injection device/s, including debris like broken cells or pieces of plaques accumulating during treatment of the targeted treatment site with the intraluminal medical device according to the invention.

The beneficial agent(s) is either delivered by the injection device and/or the beneficial agent lumen according to the present invention. Both the injection device and the beneficial agent lumen constrain the beneficial agent within the housing area. Because the inner surface of the body lumen defines an exterior boundary to the housing area, beneficial agent(s) contained within the housing area are maintained against the surface being treated. Thus, the intraluminal medical treatment device according to the present invention targets the beneficial agent(s) to a specific site without interference from fluid flow in the lumen.

Examples of potential beneficial agents that may be used include, but are not limited to, pharmacologic agents, anti-platelet agents, anti-inflammatory agents, anti-thrombotic agents, thrombolytic agents, and/or immunosuppressant agents.

The pharmacologic agents that can be effective in preventing restenosis can be classified into the categories of anti-proliferative agents, anti-platelet agents, anti-inflammatory agents, anti-thrombotic agents, and thrombolytic agents. Anti-proliferative agents may include, for example, crystalline rapamycin. These classes can be further subdivided. For example, anti-proliferative agents can be anti-mitotic. Anti-mitotic agents inhibit or affect cell division, whereby processes normally involved in cell division do not take place. One sub-class of anti-mitotic agents includes vinca alkaloids. Representative examples of vinca alkaloids include, but are not limited to, vincristine, paclitaxel, etoposide, nocodazole, indirubin, and anthracycline derivatives, such as, for example, daunorubicin, daunomycin, and plicamycin. Other sub-classes of anti-mitotic agents include anti-mitotic alkylating agents, such as, for example, tauromustine, bofumustine, and fotemustine, and anti-mitotic metabolites, such as, for example, methotrexate, fluorouracil, 5-bromodeoxyuridine, 6-azacytidine, and cytarabine. Anti-mitotic alkylating agents affect cell division by covalently modifying DNA, RNA, or proteins, thereby inhibiting DNA replication, RNA transcription, RNA translation, protein synthesis, or combinations of the foregoi ng.

Anti-platelet agents are therapeutic entities that act by (1) inhibiting adhesion of platelets to a surface, typically a thrombogenic surface, (2) inhibiting aggregation of platelets, (3) inhibiting activation of platelets, or (4) combinations of the foregoing. Activation of platelets is a process whereby platelets are converted from a quiescent, resting state to one in which platelets undergo a number of morphologic changes induced by contact with a thrombogenic surface. These changes include changes in the shape of the platelets, accompanied by the formation of pseudopods, binding to membrane receptors, and secretion of small molecules and proteins, such as, for example, ADP and platelet factor 4. Anti-platelet agents that act as inhibitors of adhesion of platelets include, but are not limited to, eptifibatide, tirofiban, RGD (Arg-Gly-Asp)-based peptides that inhibit binding to gpllbllla or avβ3, antibodies that block binding to gpIIaIIIb or avβ3, anti-P-selectin antibodies, anti-E-selectin antibodies, compounds that block P-selectin or E-selectin binding to their respective ligands, saratin, and anti-von Willebrand factor antibodies. Agents that inhibit ADP-mediated platelet aggregation include, but are not limited to, disagregin and cilostazol.

Anti-inflammatory agents can also be used. Examples of these include, but are not limited to, prednisone, dexamethasone, hydrocortisone, estradiol, fluticasone, clobetasol, and non-steroidal anti-inflammatories, such as, for example, acetaminophen, ibuprofen, naproxen, and sulindac. Other examples of these agents include those that inhibit binding of cytokines or chemokines to the cognate receptors to inhibit proinflammatory signals transduced by the cytokines or the chemokines. Representative examples of these agents include, but are not limited to, anti-IL1, anti-IL2, anti-IL3, anti-IL4, anti-IL8, anti-IL15, anti-IL18, anti-GM-CSF, and anti-TNF antibodies.

Anti-thrombotic agents include chemical and biological entities that can intervene at any stage in the coagulation pathway. Examples of specific entities include, but are not limited to, small molecules that inhibit the activity of factor Xa. In addition, heparinoid-type agents that can inhibit both FXa and thrombin, either directly or indirectly, such as, for example, heparin, heparin sulfate, low molecular weight heparins, such as, for example, the compound having the trademark Clivarin®, and synthetic oligosaccharides, such as, for example, the compound having the trademark Arixtra®. Also included are direct thrombin inhibitors, such as, for example, melagatran, ximelagatran, argatroban, inogatran, and peptidomimetics of binding site of the Phe-Pro-Arg fibrinogen substrate for thrombin. Another class of anti-thrombotic agents that can be delivered is factor VII/VIIa inhibitors, such as, for example, anti-factor VII/VIIa antibodies, rNAPc2, and tissue factor pathway inhibitor (TFPI).

Thrombolytic agents, which may be defined as agents that help degrade thrombi (clots), can also be used as adjunctive agents, because the action of lysing a clot helps to disperse platelets trapped within the fibrin matrix of a thrombus. Representative examples of thrombolytic agents include, but are not limited to, urokinase or recombinant urokinase, pro-urokinase or recombinant pro-urokinase, tissue plasminogen activator or its recombinant form, and streptokinase.

One or more immunosuppressant agents may be used. Immunosuppressant agents may include, but are not limited to, IMURAN® azathioprine sodium, brequinar sodium, SPANIDIN® gusperimus trihydrochloride (also known as deoxyspergualin), mizoribine (also known as bredinin), CELLCEPT® mycophenolate mofetil, NEORAL® Cylosporin A (also marketed as different formulation of Cyclosporin A under the trademark SANDIM-MUNE®), PROGRAF® tacrolimus (also known as FK-506), sirolimus and RAPAMUNE®, leflunomide (also known as HWA-486), glucocorticoids, such as prednisolone and its derivatives, antibody therapies such as orthoclone (OKT3) and Zenapax®, and antithymyocyte globulins, such as thymoglobulins. In addition, a crystalline rapamycin analog, A-94507, SDZ RAD (a.k.a. Everolimus), and/or other immunosuppressants.

The beneficial agents may also include stem cells, tumor treating seeds, coagulants, anticoagulants, microspheres, radioactive materials, and/or other similar agents. In a yet further configuration, the beneficial agents may include a resin that may be activated or cured by an activator, such as ultraviolet light, to form a solidified substance.

In one embodiment the interluminal medical treatment device according to the present invention further comprises at least one other medical device or endoprosthesis. In a preferred embodiment the medical device is selected from the group of stent, stent-graft, implant, balloon or filter-devices; preferably is a stent, especially a balloon expandable or self-expanding stent, or is a balloon. Preferably, the at least one other medical device is positioned on the surface of the outer member and between the proximal and distal portion of the outer membrane or is disposed circumferentially outside the outer member and between the proximal and distal portion of the outer member. Most preferably, the at least one other medical device is a stent, wherein the stent is a self-expanding stent or a balloon expandable stent. Preferably the at least one other medical device is made of a self-expanding material or shape memory material, preferably is a self-expanding stent.

Most preferably, the surface of at least one other medical device, e.g. a stent, is suitable to be loaded with at least one beneficial agents. This has the advantage that the stent can be loaded with beneficial agent(s) after the stent has been positioned within the lumen at the site of treatment. Such advantage is in particular desired for self-expandable stents. As it is known from the prior art, self-expandable stents being formed from materials such as Nitinol may loose preloaded agent during expansion. Thus, loading the expanded stent within the housing area would either allow the production or- at a later time - replenishment of a depot directly at the site of treatment after the stent was deployed. Further this has the advantage that a beneficial agent, e.g. those used to stop restenosis and thus loaded on a drug-eluting stent like rapamycin etc. (see above), could be used - if injected into the housing area - to treat the targeted site of the stent delivery in a high concentration before being removed by the beneficial agent lumen or delivery device or released into the blood flow followed by the depot effect of the drug-loaded stent. This should have a highly beneficial effect on restenosis.

In one embodiment of the interluminal medical treatment device according to the present invention the at least one other medical device is a balloon, a balloon-expandable stent or a balloon-expandable stent graft, wherein further the inner member comprises an inflation lumen as additional lumen. If combined with an injection device or devices if using the balloon e.g. for a PTA the material accumulating in the housing area may be removed from the housing area by use of the injection device/s, including debris like broken cells or pieces of plaques accumulating during treatment of the targeted treatment site with the intraluminal medical device according to the invention:

A method for treating a targeted treatment site within a lumen comprises the steps of deploying a medical device according to the present invention within the lumen proximate the targeted treatment site, and introducing at least one beneficial agent into the housing area. Preferably, the step of deploying is accompanied by pushing the retrieval member in distal direction. This method might also be used to target cancer cells or tumours being situated on the walls of body lumens like the intestine etc. allowing for the treatment (with the beneficial agent being an anticancer agent) in situ with a minimal invasive approach.

In one example the method for treating a targeted treatment site within a lumen further comprises the step of redirecting fluid flow within the lumen through an elongated inner member disposed within the outer member and at least one membrane. Preferably, the step of redirecting is performed by redirecting fluid flow into at least one perfusion port in a proximal portion of the inner member, through a perfusion lumen of the inner member, and out of at least one perfusion exit in a distal portion of the inner member.

In another example the method for treating a targeted treatment site within a lumen further comprises the step of retrieving the guidewire prior the at least one perfusion port. Retrieving of the guidewire has the advantage that the size of the perfusion lumen will be expanded if the guidewire lumen is joined to the perfusion lumen resulting in enhanced perfusion of the fluid through the inner member. Therefore, the build-up of pressure within the vessel will be prevented.

In one example the method for treating a targeted treatment site within a lumen further comprises the step of placing a stent at the targeted treatment site within the housing area.

Another example of the method for treating a targeted treatment site within a lumen further comprises introducing at least one beneficial agent into the housing area through an injection device extending at least partially into the housing area and/or the step introducing at least one beneficial agent into the housing area through a beneficial agent lumen disposed within the inner member, the beneficial agent lumen being in fluid communication with the housing area.

In one example the method for treating a targeted treatment site further comprises the step of removing plaques of the targeted treatment site within the housing area. Thereby, the plaques are treated in order to be solved and cell debris are collected by the at least one membrane and retrieved by the retrieval member within the delivery device or collected in the housing area and removed through the injection device or beneficial agent lumen. Therefore, this method allows the application of a stent at sites of very fragile plaque which usually are not properly treatable by standard methods like PTA due to the risk of the breaking away. Alternatively, it might be also possible to calcify the fragile plaques present within the housing area using beneficial agents, making them more susceptible to standard treatment.

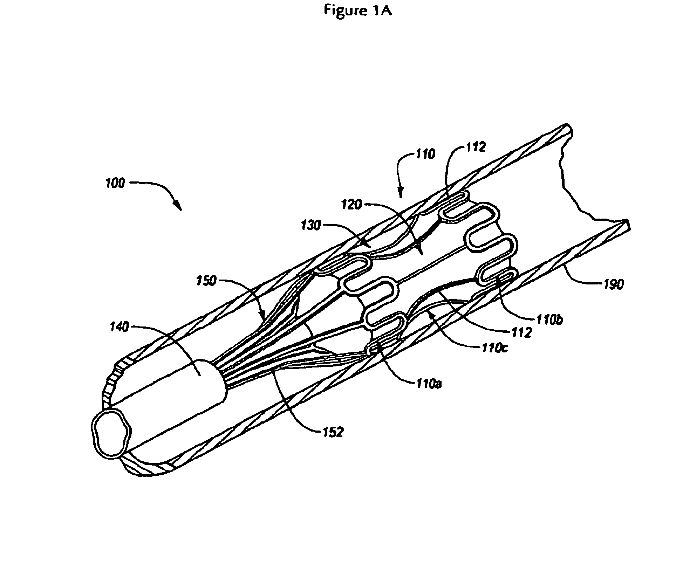

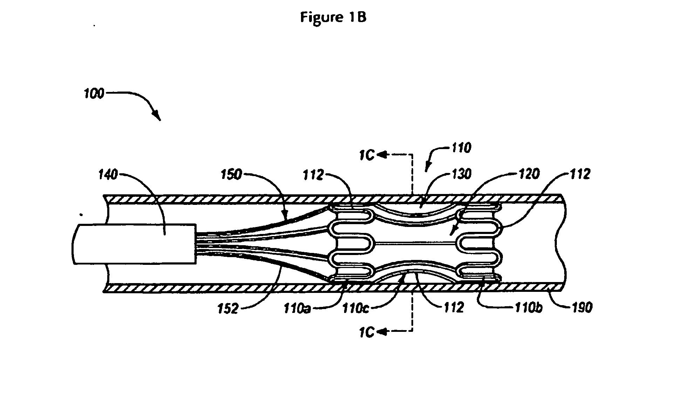

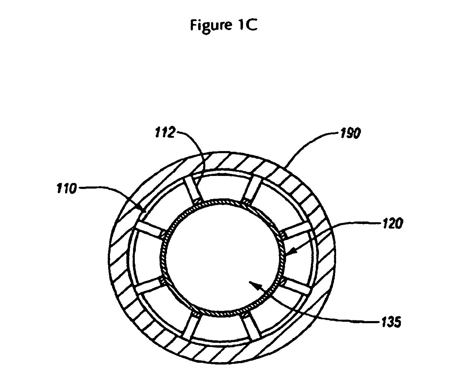

Reference is now made to FIGS. 1A-1C, which illustrate an example interluminal medical treatment device 100 deployed within a body lumen 190 in accordance with an implementation of the present disclosure. Specifically, FIG. 1A illustrates a perspective view of the interluminal medical treatment device 100 in a deployed position within the body lumen 190; FIG. 1B illustrates a side view of the interluminal medical treatment device 100 of FIG. 1A; and FIG. 1C illustrates a cut away view of the interluminal medical treatment device 100 of FIG. 1B along the line 1C-1C.

As disclosed in FIGS. 1A-1C, the interluminal medical treatment device 100 may include an outer member 110 having an expandable proximal portion 110a, an expandable distal portion 110b, and a central portion 110c of reduced expandability. In other words, the central portion 110c may have a smaller expanded outside diameter than the expanded outside diameters of the expandable proximal portion 110a and expandable distal portion 110b. The interluminal medical treatment device may also have a membrane 120 coupled to the outer member 110.

When in an expanded position, the outer member 110 and membrane 120 may define a housing area 130, into which one or more beneficial agents can be introduced. In addition, the interluminal medical treatment device 100 may include a retrieval member 150 coupled to the proximal portion 110a of the outer member 110. The interluminal medical treatment device 100 may be retrieved and/or deployed using a delivery device 140, such as a catheter.

In an embodiment, the interluminal medical treatment device 100 may be disposed within the delivery device 140 in a collapsed position. The delivery device 140 may then deliver the interluminal medical treatment device 100 near a targeted treatment site within a body lumen 190. The retrieval member 150 can then be used to deploy the interluminal medical treatment device 100. In particular, pushing the retrieval member 150 in a distal direction can force the interluminal medical treatment device 100 out of the delivery device 140. Upon leaving the delivery device 140, the outer member 110 and membrane 120 may unfold and/or expand into a deployed position, thereby engaging the inner wall of the body lumen 190 and defining a housing area 130 proximate the targeted treatment site. Beneficial agent(s) may then be introduced into the housing area 130 to treat the targeted treatment site.

In one implementation, beneficial agent(s) may be introduced into the housing area 130 while the interluminal medical treatment device 100 is being deployed from its delivery device 140. For example, an injection device, such as an injection catheter or needle, can introduce beneficial agent(s) into the housing area 130 once the distal portion 110b is deployed and while the proximal portion 110a remains in a collapsed position, such as within the delivery device 140. In a further implementation, injections of beneficial agent(s) into the housing area 130 may be completed after full deployment of the interluminal medical treatment device 100.

Once the interluminal medical treatment device 100 is deployed, the membrane 120 may redirect fluid within the body lumen 190 away from the housing area 130 and through an inner passageway 135 defined by the inside surface of the membrane 120 and/or outer member 110, thereby at least partially isolating the housing area 130 from fluid flow within the body lumen 190. As a result, beneficial agent(s) within the housing area 130 may be at least partially protected from being carried away from the targeted treatment site by fluid flow within the body lumen 190. Accordingly, treatment of a targeted treatment site within a body lumen 190 may be protected and improved.

Once treatment is completed, the interluminal medical treatment device 100 may be removed from the body lumen 190. For example, by pulling the retrieval member 150 in a proximal direction relative to the delivery device 140, the interluminal medical treatment device 100 may be collapsed and pulled into the delivery device 140. Thereafter, the interluminal medical treatment device 100 may be completely retrieved from the body.

The outer member 110 of the interluminal medical treatment device 100 may include an expandable implantable medical device or endoprosthesis, such as a stent, filter, or other endoprosthesis. The outer member 110 may be self-expanding or balloon expandable. As mentioned, the outer member 110 may include an expandable proximal portion 110a, an expandable distal portion 110b, and a central portion 110c of relatively lower expandability as compared to the proximal portion 110a and distal portion 110b. As shown in FIGS. 1A-1C, the outer member 110 may be formed using a plurality of struts 112. In particular, the proximal portion 110a and distal portion 110b may each include an expandable ring of struts 112. For example, the struts 112 of the proximal portion 110a and distal portion 110b may form a ring having a wave pattern. The central portion may include a plurality of elongated, c-shaped, and/or semi-circular struts 112 connecting the proximal portion 110a and the distal portion 110b.

In further implementations, the number and configuration of the struts may vary as desired. For example, the proximal portion 110a or distal portion 110b may each include a plurality of expandable rings or similar structures. Furthermore, the central portion 110c may include one or more rings having a lower expandability than the rings of the proximal portion 110a and distal portion 110b. The central portion 110c includes a plurality of v-shaped struts that angle inward toward the middle of the central portion 110c. In an even further embodiment, the outer member 110 can be formed using any expandable, generally-tubular body having a central portion 110c with a smaller expanded outside diameter than the proximal portion 110a and distal portion 110b.

The outer member 110 may be formed from various materials including shape memory materials, such as nickel titanium and/or alloys thereof, copper-aluminum-nickel and/or alloys thereof, copper chromium and/or alloys thereof, as well as stainless steel, other metals, polymers, biodegradable materials, bioresorbable materials, bioabsorbable materials, other similar materials, and/or combinations thereof. In addition, one or more beneficial agents or radio-opaque markers may be incorporated into the material of and/or coated over at least a portion of the outer member 110.

The proximal portion 110a and distal portion 110b of the example outer member 110 of FIGS. 1A-1C may have a generally tubular or cylindrical shape. However, in further configurations, the proximal portion 110a and distal portions 110b of the outer member 110 may incorporate any expanded shape capable of engaging an inner wall of the body lumen 190. For example, the proximal portion 110a or distal portion 110b may have a spherical shape, conical shape, diamond shape, spheroidal shape, and/or other similar shapes.

As mentioned, the example interluminal medical treatment device 100 of FIGS. 1A-1C may also include a membrane 120. The membrane 120 may be disposed within and coupled to an inner surface of the outer member 110. In further configurations, the membrane 120 may be disposed outside and coupled to an outer surface of the outer member 110.

The example membrane 120 may extend along the central portion 110c of the outer member 110 and at least partially along the proximal portion 110a and distal portion 110b. In further configurations, however, the membrane 120 may extend further or lesser along the portions 110a, 110b, 110c of the outer member 110. In addition, although the example membrane 120 of FIGS. 1A-1C is a single piece, in still further configurations, the membrane 120 may comprise a plurality of pieces coupled to the outer member 110.

The membrane 120 can incorporate any porosity desired for a particular configuration. For example, the membrane 120 can be substantially permeable, semi-permeable, impermeable, substantially porous, or non-porous. The porosity or permeability of the membrane 120 may be adjusted as desired depending on the type of beneficial agent to be administered and the desired treatment. For example, a porous or permeable material may be used for the membrane 120 if a residual flow through the membrane 120 is desired. Furthermore, the porosity or permeability of the membrane 120 may vary from one portion of the membrane 120 to another.

The membrane 120 may comprise any of a number of different materials. For example, the membrane 120 may include polymers, such as plastics, polyurethanes, synthetic fabrics, elastomers, and/or other similar materials. In one example embodiment, the membrane 120 may comprise a polymer film. Examples of the material to be used in the membrane include polymers, polyurethanes, synthetic fabrics, elastomers, polyamides, silicone, PTFE, and/or ePTFE. In a further embodiment, the membrane 120 may comprise one or more balloons, such as balloons configured to expand the outer member 110 and/or drug eluting balloons.

In a yet further embodiment, the membrane 120 may be integral with the outer member 110. For example, the membrane 120 and outer member 110 may be of a unitary construct. In particular, the membrane 120 may be formed with and be an integral part of the outer member 110.

As introduced, the outer member 110 and/or membrane 120 may define a housing area 130 extending annularly around the interluminal medical treatment device 100. In one implementation, the housing area 130 may be located proximate the central portion 110c and between the proximal portion 110a and distal portion 110b. As a result, when the interluminal medical treatment device 100 is deployed within a body lumen 190, as shown in FIGS. 1A-1C, the outer member 110 and membrane 120 may redirect fluid flow within the body lumen 190 away from the housing area 130 and through an inner passageway 135 defined by the inside surface of the membrane 120. Accordingly, the interluminal medical treatment device 100 may at least partially isolate and protect the contents of the housing area 130, while maintaining fluid flow within the body lumen 190. In additional configurations, the outer member 110 and membrane 120 may define multiple housing areas 130 or the housing area 130 may extend around only a portion of the body lumen 190. In a yet further configuration, the outer member 110 and membrane 120 may be deployed so as to contain a targeted treatment site within the housing area 130 without exerting physical forces directly onto the targeted treatment site.

As mentioned above, the interluminal medical treatment device 100 may also include a retrieval member 150 configured to retrieve and/or deploy the interluminal medical treatment device 100. As shown in FIGS. 1A-1C, the retrieval member 150 may be coupled to the outer member 110, such as to the proximal portion 110a. The retrieval member 150 may also be configured to at least partially extend into the delivery device 140. For example, the retrieval member 150 may include a plurality of elongated struts 152 coupled to the proximal portion 110a and configured to extend at least partially into the delivery device 140. In further configurations, the retrieval member 150 may include additional structural elements to assist in retrieving and/or deploying the interluminal medical treatment device 100, such as a hook or other similar elements.

Pushing the retrieval member 150 in a distal direction relative to the delivery device 140 may force the interluminal medical treatment device 100 out of the delivery device 140 and into a deployed position. Alternatively, pulling the retrieval member 150 in a proximal direction relative to the delivery device 140 can force the interluminal medical treatment device 100 into the delivery device 140 and into a collapsed position.

As introduced above, once the interluminal medical treatment device 100 is deployed or is in the process of being deployed proximate a targeted treatment site, one or more beneficial agents may be introduced into the housing area 130. Because the inner surface of the body lumen 190 defines an exterior boundary to the housing area 130, beneficial agent(s) contained within the housing area 130 may be maintained against the surface being treated. The membrane 120 and/or outer member 110 may also protect the beneficial agent(s) from fluid flow within the body lumen 190 that would otherwise dilute the beneficial agent(s) or carry the beneficial agent(s) away from the targeted treatment site. As a result, treatment of a targeted site within the body lumen 190 may be accomplished in a more precise, controlled, and efficient manner.

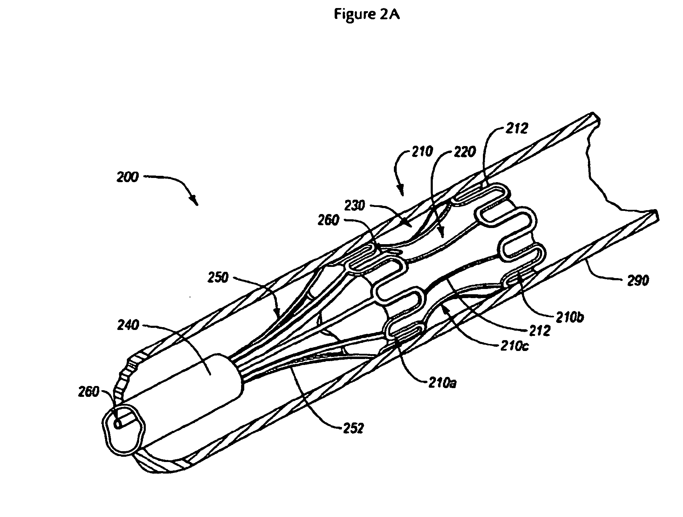

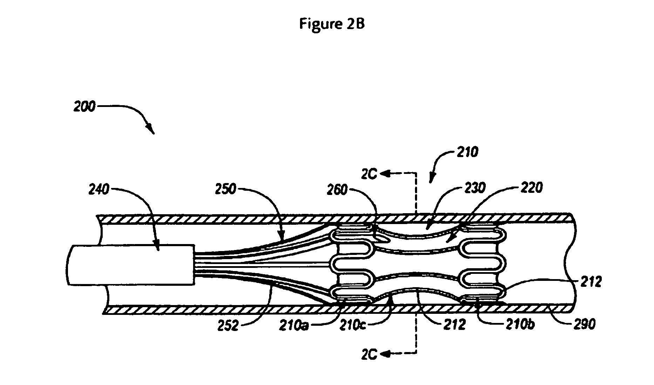

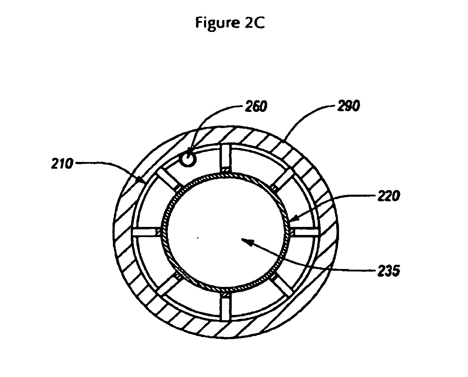

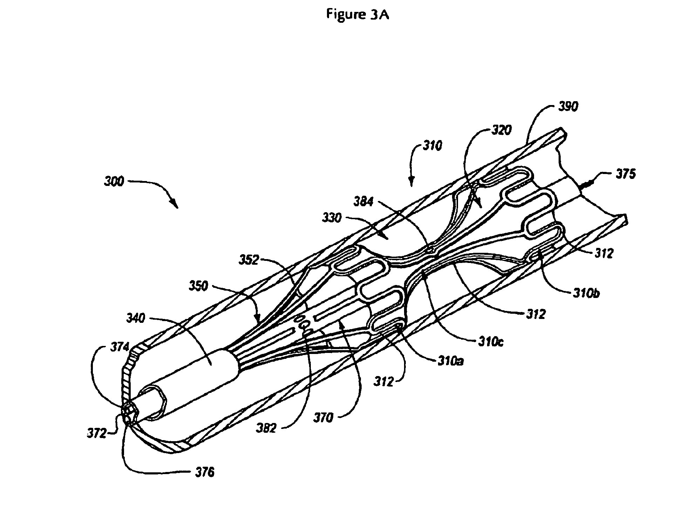

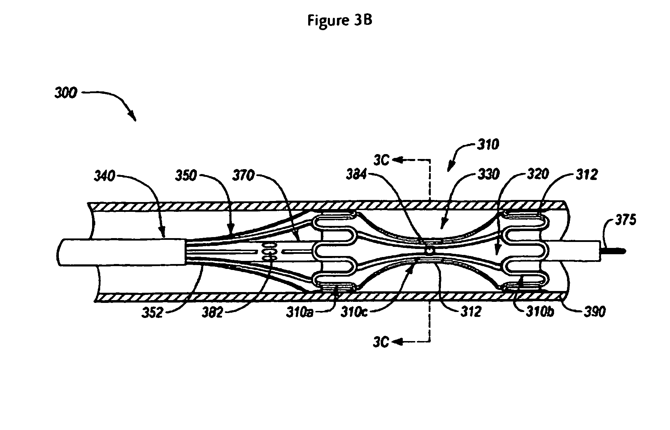

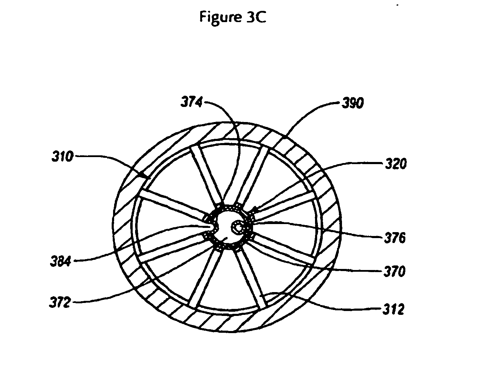

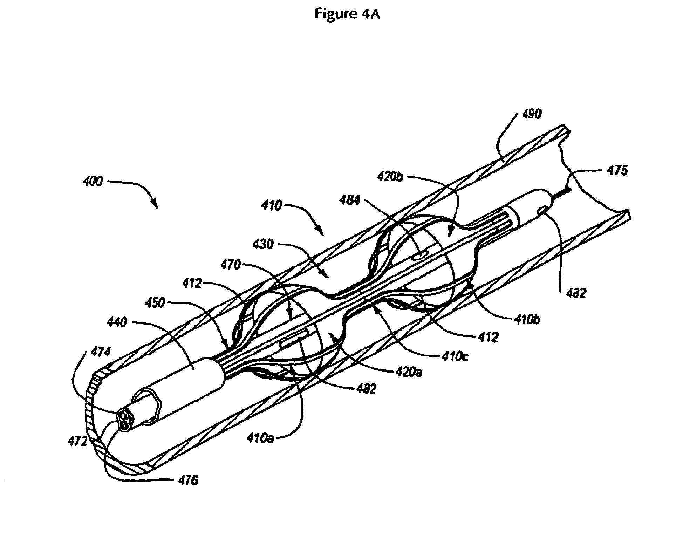

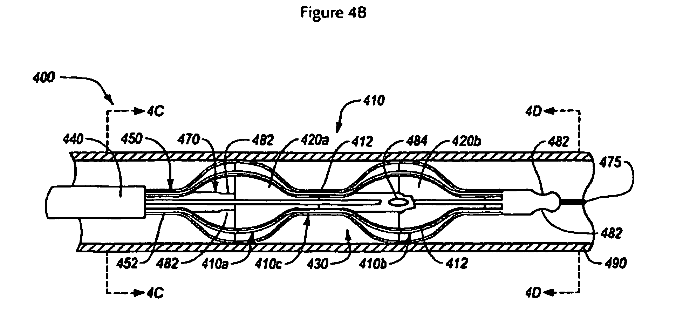

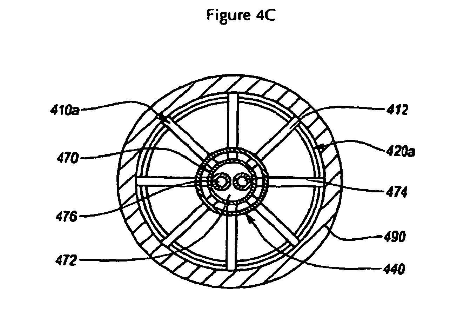

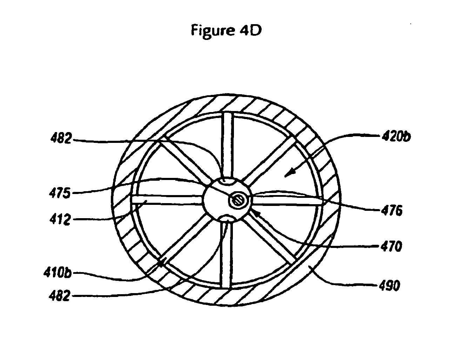

The interluminal medical treatment device 100 may incorporate at least one component of the interluminal medical treatment devices 200, 300, 400, 500, and 600 described in connection with FIGS. 2-6, respectively. The following non-limiting list of examples indicates the interchangeability of at least some of the components of the interluminal medical treatment devices 100, 200, 300, 400, 500, and 600 described herein. For instance, the interluminal medical treatment device 100 may include one or more injection devices (e.g., 260, as shown in FIGS. 2A-2C). In another example, the interluminal medical treatment device 100 may further incorporate an inner member (e.g., 370 and 470, as shown in FIGS. 3 and 4, respectively) disposed within the outer member 110 and membrane 120.

Reference is now made to FIGS. 2A-2C, which disclose another example interluminal medical treatment device 200 in accordance with an implementation of the present disclosure. Specifically, FIG. 2A illustrates a perspective view of the interluminal medical treatment device 200 in a deployed position within a body lumen 290; FIG. 2B illustrates a side view of the interluminal medical treatment device 200 of FIG. 2A; and FIG. 2C illustrates a cut away view of the interluminal medical treatment device 200 of FIG. 2B along the line 2C-2C.

The example interluminal medical treatment device 200 of this configuration may be functionally similar to the example interluminal medical treatment device 100 previously described above and shown in FIGS. 1A-1C in most respects, wherein certain features will not be described in relation to this configuration wherein those components may function in the manner as described above and are hereby incorporated into this additional configuration described below. Like structures and/or components are given like reference numerals. Additionally, the interluminal medical treatment device 200 may incorporate at least one component of the interluminal medical treatment devices 100, 300, 400, 500, and 600 described in connection with FIGS. 1, and 3-6, respectively.

As disclosed in FIGS. 2A-2C, the interluminal medical treatment device 200 may include an outer member 210 having an expandable proximal portion 210a, an expandable distal portion 210b, and a central portion 210c of reduced expandability. The interluminal medical treatment device may also have a membrane 220 coupled to the outer member 210. When in an expanded position, the outer member 210 and membrane 220 may define a housing area 230, into which one or more beneficial agents may be introduced. The interluminal medical treatment device 200 may also include an injection device 260, such as a needle and/or catheter, disposed through the delivery device 240 and extending at least partially into the housing area 230. In addition, the interluminal medical treatment device 200 may include a retrieval member 250 coupled to the outer member 210. The interluminal medical treatment device 200 may be retrieved and/or deployed using a delivery device 240, such as a catheter.

In one embodiment, the interluminal medical treatment device 200 may be disposed within the delivery device 240 in a collapsed position. The delivery device 240 may then deliver the interluminal medical treatment device 200 near a targeted treatment site within a body lumen 290. The retrieval member 250 may then be used to deploy the interluminal medical treatment device 200. In particular, pushing the retrieval member 250 in a distal direction may move the interluminal medical treatment device 200 out of the delivery device 240. Upon leaving the delivery device 240, the outer member 210 and membrane 220 may unfold and/or expand into a deployed position, thereby engaging the inner wall of the body lumen 290 and defining a housing area 230 proximate a targeted treatment site. Using the injection device 260, beneficial agent(s) may then be introduced into the housing area 230 to treat the targeted site.

Once the interluminal medical treatment device 200 is deployed, the membrane 220 and/or outer member 210 may redirect fluid within the body lumen 290 away from the housing area 230 and through an inner passageway 235 defined by the inside surface of the membrane 220 and outer member 210, thereby at least partially isolating the housing area 230 from fluid flow within the body lumen 290. As a result, beneficial agent(s) within the housing area 230 may be protected from being diluted or carried away from the targeted treatment site by fluid flow within the body lumen 290.

Once treatment is completed, the interluminal medical treatment device 200 may be removed from the body lumen 290. For example, by pulling the retrieval member 250 in a proximal direction relative to the delivery device 240, the interluminal medical treatment device 200 may be collapsed and pulled into the delivery device 240 and thereafter, completely retrieved from the body.

The outer member 210 of the interluminal medical treatment device 200 may include an expandable implantable medical device or prosthesis, such as a stent, filter, or other endoprosthesis. The outer member 210 may include an expandable proximal portion 210a, an expandable distal portion 210b, and a central portion 210c of reduced expandability. In one example configuration, the outer member 210 may be formed using a plurality of struts 212. In particular, the proximal portion 210a and distal portion 210b may each include an expandable ring of struts 212. The central portion may include a plurality of elongated struts 212 connecting the proximal portion 210a and the distal portion 210b. In particular, the elongated struts 212 of the central portion 210c may taper and/or curve radially inward from the proximal portion 210a and distal portion 210b to the middle of the central portion 210c. As a result, the deployed position of the central portion 210c may have a smaller diameter than the proximal portion 210a or distal portion 210b.

In further implementations, the number and configuration of the struts may vary as desired. For example, the proximal portion 210a or distal portion 210b may each include a plurality of expandable rings or similar structures. Furthermore, the central portion 210c can include one or more rings having a lesser expandability than the rings of the proximal portion 210a and distal portion 210b. In a further embodiment, the outer member 210 can be formed using any expandable, generally-tubular body.

The outer member 210 may be formed from various materials including shape memory materials, such as nickel titanium and/or alloys thereof, copper-aluminum-nickel and/or alloys thereof, copper chromium and/or alloys thereof, as well as stainless steel, other metals, polymers, biodegradable materials, bioresorbable materials, bioabsorbable materials, other similar materials, and/or combinations thereof. In addition, one or more beneficial agents may be incorporated into the material of and/or coated over at least a portion of the outer member 210.

The proximal portion 210a and distal portion 210b of the example outer member 210 of FIGS. 2A-2C may have a generally tubular or cylindrical shape. However, in further configurations, the proximal portion 210a and distal portion 210b of the outer member 210 may incorporate any expanded shape capable of engaging an inner wall of the body lumen 290. For example, the proximal portion 210a or distal portion 210b may have a spherical shape, conical shape, diamond shape, spheroidal shape, and/or other similar shapes.

As mentioned, the example interluminal medical treatment device 200 of FIGS. 2A-2C also includes a membrane 220. The example membrane 220 can be disposed within and coupled to an inner surface of the outer member 210. In further configurations, the membrane 220 may be disposed outside and coupled to an outer surface of the outer member 210.

The membrane 220 can extend along the central portion 210c of the outer member 210 and at least partially along the proximal portion 210a and distal portion 210b. In a further embodiment, the membrane 220 may extend along substantially the entire length of the outer member 210, as shown in the example illustrated in FIGS. 2A-2C. In further configurations, the membrane 220 may comprise multiple pieces coupled to the outer member 210.

The membrane 220 can incorporate any porosity and/or permeability desired for a particular configuration. For example, the membrane 220 can be porous, semi-porous, impermeable, or other similar configurations. As a result, the membrane 220 can at least partially redirect fluid flow within a body lumen 290 when the interluminal medical treatment device 200 is in a deployed position.

The outer member 210 and/or membrane 220 can define a housing area 230 extending annularly around the interluminal medical treatment device 200 proximate the central portion 210c. As a result, when the interluminal medical treatment device 200 is deployed within a body lumen 290, as shown in FIGS. 2A-2C, the outer member 210 and membrane 220 can redirect fluid flow within the body lumen 290 away from the housing area 230 and through an inner passageway 235 defined by the inside surface of the membrane 220. As a result, the interluminal medical treatment device 200 can at least partially isolate and protect the contents of the housing area 230, while maintaining fluid flow within the body lumen 290. In additional configurations, the outer member 210 and membrane 220 may define multiple housing areas 230 or the housing area 230 may extend around only a portion of the interluminal medical treatment device 200.

As mentioned above, the interluminal medical treatment device 200 may also include a retrieval member 250 configured to retrieve and/or deploy the interluminal medical treatment device 200. For example, as shown in FIGS. 2A-2C, the retrieval member 250 can be coupled to the outer member 210, such as to the proximal portion 210a. The retrieval member 250 can also be configured to at least partially extend into the delivery device 240. In one configuration, the retrieval member 250 can include a plurality of elongated struts 252 coupled to the proximal portion 210a and configured to extend at least partially into the delivery device 240. In further configurations, the retrieval member 250 may include additional structural elements, such as a hook or other similar elements for retrieving and/or deploying the interluminal medical treatment device 200.

Pushing the retrieval member 250 in a distal direction relative to the delivery device 240 can force the interluminal medical treatment device 200 out of the delivery device 240 and into a deployed position. Alternatively, pulling the retrieval member 250 in a proximal direction relative to the delivery device 240 can force the interluminal medical treatment device 200 into the delivery device 240 and into a collapsed position. Accordingly, the retrieval member 250 may assist in the deployment and retrieval of the interluminal medical treatment device 200.

The interluminal medical treatment device 200 can also include an injection device 260, such as a needle and/or catheter, to introduce beneficial agent(s) into the housing area 230. In the example configuration of FIGS. 2A-2C, the injection device 260 passes through the delivery device 240 and extends at least partially into the housing area 230. The injection device 260 may extend from a point external to a patient receiving treatment through the lumen 290 and into the housing area 230. As a result, a physician may use a syringe or other external device to introduce a beneficial agent into the housing area 230 through the injection device 260. In one example embodiment, the injection device 260 can be coupled to an automated device, whether internal or external to the patient, configured to automatically introduce a predetermined amount of beneficial agent into the housing area 230 at predetermined time intervals.

In one example configuration, the injection device 260 may be temporarily positioned within the housing area 230 and then removed once a beneficial agent is introduced into the housing area 230. In a further configuration, the injection device 260 may remain within the housing area 230 for an extended period of time, thereby facilitating sustained and/or ongoing treatments of a targeted treatment site within the body lumen 290. In a yet further configuration, the injection device 260 may be connected to the outer member 210, membrane 220, and/or retrieval member 250. In additional configurations, the interluminal medical treatment device 200 may include multiple injection devices 260 extending into the housing area 230, through which the same or different beneficial agents may be introduced. Furthermore, one or more beneficial agents may be introduced through a single injection device 260.

The size of the injection device 260 may vary as necessary for a particular configuration. In one example embodiment, the injection device 260 may include a 27 gauge, or larger, needle. The injection device 260 may also be configured for the delivery of cells to the housing area 230.

Once the interluminal medical treatment device 200 is deployed proximate a targeted treatment site, one or more beneficial agents may be introduced into the housing area 230. Because the inner surface of the body lumen 290 may define an exterior boundary to the housing area 230, beneficial agent(s) contained within the housing area 230 can be maintained directly against the inner wall of the body lumen 290 being treated. The outer member 210 and membrane 220 can also protect the beneficial agent(s) from fluid flow within the body lumen 290 that would otherwise carry the beneficial agent(s) away from the targeted treatment site. Accordingly, treatment of a targeted site within the body lumen 290 can be accomplished in a more precise, controlled, and efficient manner.

Further aspects of the present disclosure are disclosed in FIGS. 3A-3C, which illustrate yet another example interluminal medical treatment device 300 in accordance with an implementation of the present disclosure. Specifically, FIG. 3A illustrates a perspective view of the interluminal medical treatment device 300 in a deployed position within a body lumen 390; FIG. 3B illustrates a side view of the interluminal medical treatment device 300 of FIG. 3A; and FIG. 3C illustrates a cut away view of the interluminal medical treatment device 300 of FIG. 3B along the line 3C-3C. The example interluminal medical treatment device 300 of this configuration may be functionally similar to the example interluminal medical treatment devices 100 and 200 previously described above and shown in FIGS. 1 and 2 in most respects, wherein certain features will not be described in relation to this configuration wherein those components may function in the manner as described above and are hereby incorporated into this additional configuration described below. Like structures and/or components are given like reference numerals. Additionally, the interluminal medical treatment device 300 may incorporate at least one component of the interluminal medical treatment devices 100, 200, 400, 500, and 600 described in connection with FIGS. 1, 2, and 4-6, respectively.

As disclosed in FIGS. 3A-3C, the example interluminal medical treatment device 300 includes an outer member 310 having an expandable proximal portion 310a, an expandable distal portion 310b, and a central portion 310c of reduced expandability. In other words, the central portion 310c may have a smaller expanded outside diameter than the expanded outside diameters of the expandable proximal portion 310a and expandable distal portion 310b. The interluminal medical treatment device may also have a membrane 320 coupled to the outer member 310.

When in an expanded position, the outer member 310 and membrane 320 may define a housing area 330, into which one or more beneficial agents may be introduced. The interluminal medical treatment device 300 may also include a generally elongated and/or tubular inner member 370 disposed within the outer member 310 and membrane 320. The inner member 370 may include a beneficial agent lumen 374, a guidewire lumen 376, and/or a perfusion lumen 372. The inner member 370 may also include one or more corresponding perfusion ports 382 and/or beneficial agent ports 384. In addition, the interluminal medical treatment device 300 can include a retrieval member 350 coupled to the proximal portion 310a of the outer member 310. Finally, the interluminal medical treatment device 300 can be retrieved and/or deployed using a delivery device 340, such as a catheter.

In one embodiment, the interluminal medical treatment device 300 can be disposed within the delivery device 340 in a collapsed position. The delivery device 340 can then deliver the interluminal medical treatment device 300 near a targeted treatment site within a body lumen 390. The retrieval member 350 can then be used to deploy the interluminal medical treatment device 300. In particular, pushing the retrieval member 350 in a distal direction can force the interluminal medical treatment device 300 out of the delivery device 340. Upon leaving the delivery device 340, the outer member 310 and membrane 320 can unfold and/or expand into a deployed position, thereby engaging the inner wall of the body lumen 390 and defining a housing area 330 proximate a targeted treatment site. Beneficial agent(s) can then be introduced into the housing area 330 via the beneficial agent lumen 374 and corresponding beneficial agent port 384 to treat the targeted treatment site.

Once the interluminal medical treatment device 300 is deployed, the membrane 320 can redirect fluid within the body lumen 390 away from the housing area 330 and through the perfusion lumen 374 of the inner member 370, thereby at least partially isolating the housing area 330 from fluid flow within the body lumen 390. As a result, beneficial agent(s) within the housing area 330 can be protected from being carried away from the targeted treatment site by fluid flow within the body lumen 390.

Once treatment is completed, the interluminal medical treatment device 300 can be removed from the body lumen 390. For example, by pulling the retrieval member 350 in a proximal direction relative to the delivery device 340, the interluminal medical treatment device 300 can be collapsed and pulled into the delivery device 340. Thereafter, the interluminal medical treatment device 300 can be completely retrieved from the body.

As mentioned, the interluminal medical treatment device 300 can include a generally tubular outer member 310, such as a stent or other endoprosthesis. The outer member 310 can include an expandable proximal portion 310a, an expandable distal portion 310b, and a central portion 310c of reduced expandability. In the example configuration of FIGS. 3A-3C, the outer member 310 may be formed using a plurality of struts 312. In particular, the proximal portion 310a and distal portion 310b may each include an expandable ring of struts 312. The central portion can include a plurality of elongated struts 312 connecting the proximal portion 310a and the distal portion 310b. In particular, the elongated struts 312 of the central portion 310c can taper and/or curve radially inward from the proximal portion 310a and distal portion 310b to the longitudinal middle of the central portion 310c. In a further configuration, the central portion 310c may be at least partially coupled to the inner member 370. As a result, the deployed position of the central portion 310c has a smaller diameter than the deployed position of the proximal portion 310a and distal portion 310b.

The outer member 310 may be formed from various materials including nickel titanium and/or alloys thereof, copper-aluminum-nickel and/or alloys thereof, copper chromium and/or alloys thereof, as well as stainless steel, polymers, biodegradable materials, bioresorbable materials, bioabsorbable materials, other similar materials, and/or combinations thereof. In addition, one or more beneficial agents may be incorporated into the material of and/or coated over at least a portion of the outer member 310.

The proximal portion 310a and distal portion 310b of the example outer member 310 of FIGS. 3A-3C can have a generally tubular or cylindrical shape. However, in further configurations, the proximal portion 310a and distal portion 310b of the outer member 310 may incorporate any expanded shape capable of engaging an inner wall of the body lumen 390. For example, the proximal portion 310a or distal portion 310b may have a spherical shape, conical shape, diamond shape, spheroidal shape, and/or other similar shapes.

As mentioned, the example interluminal medical treatment device 300 of FIGS. 3A-3C also includes a membrane 320. The membrane 320 may be disposed within and coupled to an inner surface of the outer member 310. In further configurations, the membrane 320 may be disposed outside and coupled to an outer surface of the outer member 310.

The membrane 320 can extend along the central portion 310c of the outer member 310 and at least partially along the proximal portion 310a and distal portion 310b. In a further configuration, the example membrane 320 may extend along substantially the entire length of the outer member 310, as shown in the example disclosed in FIGS. 3A-3C. In addition, the membrane 320 can comprise a plurality of pieces coupled to the outer member 310.

The membrane 320 can incorporate any porosity and/or permeability desired for a particular configuration. For example, the membrane 320 can be porous, semi-porous, impermeable, or other similar configurations. As a result, the membrane 320 can at least partially redirect fluid flow within a body lumen 390 when the interluminal medical treatment device 300 is in a deployed position.

The outer member 310 and/or membrane 320 can define a housing area 330 extending annularly around the interluminal medical treatment device 300 about the central portion 310c and longitudinally between the proximal portion 310a and distal portion 310b. As a result, when the interluminal medical treatment device 300 is deployed within a body lumen 390, as shown in FIGS. 3A-3C, the outer member 310 and membrane 320 can redirect fluid flow within the body lumen 390 away from the housing area 330 and through a perfusion lumen 372 of the inner member 370. As a result, the interluminal medical treatment device 300 can at least partially isolate and protect the contents of the housing area 330, while maintaining fluid flow within the body lumen 390. In additional configurations, the outer member 310 and membrane 320 may define multiple housing areas 330 or the housing area 330 may extend around only a portion of the interluminal medical treatment device 300.

As mentioned above, the interluminal medical treatment device 300 may also include a retrieval member 350 configured to retrieve and/or deploy the interluminal medical treatment device 300. For example, as shown in FIGS. 3A-3C, the retrieval member 350 can be coupled to the outer member 310, such as to the proximal portion 310a. The retrieval member 350 can also be configured to at least partially extend into the delivery device 340. In one configuration, the retrieval member 350 can include a plurality of elongated struts 352 coupled to the proximal portion 310a and configured to extend at least partially into the delivery device 340. In further configurations, the retrieval member 350 may include additional structural elements, such as a hook or other similar elements.

Pushing the retrieval member 350 in a distal direction relative to the delivery device 340 can force the interluminal medical treatment device 300 out of the delivery device 340 and into a deployed position. Alternatively, pulling the retrieval member 350 in a proximal direction relative to the delivery device 340 can force the interluminal medical treatment device 300 into the delivery device 340 and into a collapsed position. Accordingly, the retrieval member 350 may assist in the deployment and retrieval of the interluminal medical treatment device 300.

As introduced above, the interluminal medical treatment device 300 can also include an inner member 370 extending longitudinally along the length of the interluminal medical treatment device 300 and being disposed within the membrane 320 and/or outer member 310. In particular, the inner member 370 can be coupled to an inner surface of the membrane 320 and/or outer member 310. The inner member 370 may comprise any of a number of different materials such as metals, plastics, and/or similar materials. Furthermore, the materials used to form the inner member 370 may be flexible or rigid.

The inner member 370 may define and/or contain one or more lumens. For example, the inner member 370 may include a perfusion lumen 372, a beneficial agent lumen 374, and/or a guidewire lumen 376. As shown in the example configuration, the beneficial agent lumen 374 and/or guidewire lumen 376 can be disposed within the perfusion lumen 372.

The inner member 370 may include one or more perfusion ports 382 in fluid communication with the perfusion lumen 372. For example, the inner member may include one or more perfusion ports 382 proximal of the housing area 330 to allow fluid flow within the body lumen 390 that has been redirected by the membrane 320 and/or outer member 310 to enter into and travel through the perfusion lumen 372 of the inner member 370. The inner member 370 may also include one or more perfusion ports 382 distal of the housing area 330 to allow fluid flow to exit the perfusion lumen 372. As a result, the housing area 330 can be isolated and protected as fluid flow within the body lumen 390 is redirected through the inner member 370.

The inner member 370 can include one or more beneficial agent ports 384 in fluid communication with the beneficial agent lumen 374 and extending into the housing area 330. Once the interluminal medical treatment device 300 is deployed, one or more beneficial agents can be introduced into the housing area 330 via the beneficial agent lumen 374 and corresponding beneficial agent port(s) 384. As a result, the beneficial agent(s) can be used to treat a targeted treatment site within the body lumen 390. In a further configuration, the inner member 370 may include a plurality of beneficial agent lumens 374 with corresponding beneficial agent ports 384 extending into the housing area 330 for the delivery of a plurality of distinct beneficial agents into the housing area 330.

The beneficial agent lumen 374 may extend from a point external to a patient receiving treatment through the lumen 390 and into the housing area 330. As a result, a physician may use a syringe or other external device to introduce a beneficial agent into the housing area 330 through the beneficial agent lumen 374. In one example embodiment, the injection device 360 can be coupled to an automated device, whether internal or external to the patient, configured to automatically inject a predetermined amount of beneficial agent into the housing area 330 at predetermined time intervals.

The inner member 370 may include a guidewire lumen 376 extending longitudinally therein. A corresponding guidewire 375 may pass through the guidewire lumen 376 and be used to position the interluminal medical treatment device 300 proximate a targeted treatment site within the body lumen 390. Once the interluminal medical treatment device 300 is positioned and/or deployed, the guidewire 375 may be removed from or remain within the guidewire lumen 376.

Reference is now made to FIGS. 4A-4C, which disclose a further example interluminal medical treatment device 400 in accordance with an implementation of the present disclosure. Specifically, FIG. 4A illustrates a perspective view of the interluminal medical treatment device 400 in a deployed position within a body lumen 490; FIG. 4B illustrates a side view of the interluminal medical treatment device 400 of FIG. 4A; FIG. 4C illustrates a cut away view of the interluminal medical treatment device 400 of FIG. 4B along the line 4C-4C; and FIG. 4D illustrates a cut away view of the interluminal medical treatment device 400 of FIG. 4B along the line 4D-4D.

The example interluminal medical treatment device 400 of this configuration may be functionally similar to the example interluminal medical treatment devices 100, 200, and 300 previously described above and shown in FIGS. 1-3 in most respects, wherein certain features will not be described in relation to this configuration wherein those components may function in the manner as described above and are hereby incorporated into this additional configuration described below. Like structures and/or components are given like reference numerals. Additionally, the interluminal medical treatment device 400 may incorporate at least one component of the interluminal medical treatment devices 100, 200, 300, 500, and 600 described in connection with FIGS. 1-3 and 5-6, respectively.

As disclosed in FIGS. 4A-4C, the example interluminal medical treatment device 400 may include an outer member 410 having an expandable proximal portion 410a, an expandable distal portion 410b, and a central portion 410c of reduced expandability. In other words, the central portion 410c may have a smaller expanded outside diameter than the expanded outside diameters of the expandable proximal portion 410a and expandable distal portion 410b. The interluminal medical treatment device may also include a proximal membrane 420a disposed within the proximal portion 410a and a distal membrane 420b disposed within the distal portion 410b. When in an expanded position, the outer member 410 and membranes 420a, 420b define a housing area 430, into which one or more beneficial agents can be introduced. The interluminal medical treatment device 400 can include an inner member 470 disposed within the outer member 410 and/or membranes 420a, 420b. The inner member 470 can include a beneficial agent lumen 474, a guidewire lumen 476, and/or a perfusion lumen 472. The inner member 470 may also include one or more corresponding perfusion ports 482 and/or beneficial agent ports 484. In addition, the interluminal medical treatment device 400 can include a retrieval member 450 coupled to the proximal portion 410a of the outer member 410. Finally, the interluminal medical treatment device 400 can be retrieved and/or deployed using a delivery device 440, such as a catheter.

In a configuration, the interluminal medical treatment device 400 can be disposed within the delivery device 440 in a collapsed position. The delivery device 440 can then deliver the interluminal medical treatment device 400 near a targeted treatment site within a body lumen 490. The retrieval member 450 can then be used to deploy the interluminal medical treatment device 400. In particular, pushing the retrieval member 450 in a distal direction can force the interluminal medical treatment device 400 out of the delivery device 440.

Upon leaving the delivery device 440, the outer member 410 and membranes 420a, 420b can unfold and/or expand into a deployed position, thereby engaging the inner wall of the body lumen 490 and defining a housing area 430 proximate a targeted treatment site. Beneficial agent(s) can then be introduced into the housing area 430 via the beneficial agent lumen 474 and corresponding beneficial agent port 484 to treat the targeted treatment site.

Once the interluminal medical treatment device 400 is deployed, the membranes 420a, 420b can redirect fluid within the body lumen 490 away from the housing area 430 and through the perfusion lumen 474 of the inner member 470, thereby at least partially isolating and protecting the housing area 430 from fluid flow within the body lumen 490. As a result, beneficial agent(s) within the housing area 430 can be protected from being diluted and/or carried away from the targeted treatment site by fluid flow within the body lumen 490.

Once treatment is completed, the interluminal medical treatment device 400 can be removed from the body lumen 490. For example, by pulling the retrieval member 450 in a proximal direction relative to the delivery device 440, the interluminal medical treatment device 400 can be collapsed and pulled into the delivery device 440. Thereafter, the interluminal medical treatment device 400 can be completely retrieved from the body.

The outer member 410 of the interluminal medical treatment device 400, may include may include an expandable endoprosthesis, such as a stent, filter, or other endoprosthesis. The outer member 410 can include an expandable proximal portion 410a, an expandable distal portion 410b, and a central portion 410c of reduced expandability. In one example configuration, the outer member 410 may be formed using a plurality of struts 412. In particular, the example outer member 410 may formed using a plurality of elongated struts 412 that extend longitudinally from the proximal portion 410a to the distal portion 410b. The struts 412 may curve, taper, and/or bend to form the proximal portion 410a and distal portion 410b with greater expanded diameters than the central portion 410c. For example, the struts may be c-shaped, v-shaped, rectangularly shaped, other similar shapes, and/or combinations of the same shapes, to provide the desired dimensions to the proximal portion 410a, distal portion 410b, and central portion 410c. In one example configuration, the elongated struts 412 may be radially spaced apart and coupled together only at the ends of the interluminal medical treatment device 400.

The outer member 410 may be formed from various materials including nickel titanium and/or alloys thereof, copper-aluminum-nickel and/or alloys thereof, copper chromium and/or alloys thereof, stainless steel, polymers, biodegradable materials, bioresorbable materials, bioabsorbable materials, other similar materials, and/or combinations thereof. In addition, one or more beneficial agents may be incorporated into the material of and/or coated over at least a portion of the outer member 410.

The proximal portion 410a and distal portion 410b of the example outer member 410 of FIGS. 4A-4C can have a generally spherical expanded shape. However, in further configurations, the proximal portion 410a and distal portions 410b of the outer member 410 may incorporate any expanded shape capable of engaging an inner wall of the body lumen 490. For example, the proximal portion 410a or distal portion 410b may have a cylindrical shape, conical shape, diamond shape, spheroidal shape, and/or other similar shapes.

As mentioned, the example interluminal medical treatment device 400 may include membranes 420a, 420b coupled to the outer member 410 and/or inner member 470. In particular, the proximal membrane 420a can be coupled to the proximal portion 410a of outer member 410. In addition, the distal membrane 420b may be coupled to the distal portion 410b of outer member 410.

The membranes 420a, 420b can be coupled to the inner member 470 and extend at least partially along the proximal portion 410a and distal portion 410b, respectively. In particular, the membranes 420a, 420b may each be umbrella-shaped extending approximately half way into the proximal and distal portions 410a, 410b, respectively. In further configurations, the membranes 420a, 420b may extend along substantially the entire length of the proximal portion 410a and distal portion 410b, respectively. Although a plurality of membranes 420a, 420b are disclosed, in a further implementation, the interluminal medical treatment device 400 may include a single membrane that extends along the proximal portion 410a, distal portion 410b, and/or central portion 410c.

The membranes 420a, 420b can incorporate any porosity and/or permeability desired for a particular configuration. For example, the membranes 420a, 420b can be porous, semi-porous, impermeable, or other similar configurations. In addition, the proximal membrane 420a can have a greater or lesser permeability and/or porosity than the distal membrane 420b. The porosity or permeability of the membrane 420 may be adjusted as desired depending on the type of beneficial agent to be administered and the desired treatment. For example, a porous or permeable material may be used for the membrane 420 if a residual flow through the membrane 420 is desired. Furthermore, the porosity or permeability of the membrane 420 may vary from one portion of the membrane 420 to another.

The outer member 410 and/or membranes 420a, 420b can define a housing area 430 extending annularly around the interluminal medical treatment device 400 about the central portion 410c and longitudinally between the proximal portion 410a and distal portion 410b. As a result, when the interluminal medical treatment device 400 is deployed within a body lumen 490, as shown in FIGS. 4A-4C, the outer member 410 and membranes 420a, 420b can redirect fluid flow within the body lumen 490 away from the housing area 430 and through a perfusion lumen 472 of the inner member 470. As a result, the interluminal medical treatment device 400 can at least partially isolate and protect the contents of the housing area 430, while maintaining fluid flow within the body lumen 490. In additional configurations, the outer member 410 and membranes 420a, 420b may define multiple housing areas 430 or the housing area 430 may extend around only a portion of the interluminal medical treatment device 400.

As mentioned above, the interluminal medical treatment device 400 may include a retrieval member 450 configured to retrieve and/or deploy the interluminal medical treatment device 400. For example, as shown in FIGS. 4A-4C, the retrieval member 450 can be coupled to the outer member 410, such as to the proximal portion 410a. The retrieval member 450 can also be configured to at least partially extend into the delivery device 440. In one configuration, the retrieval member 450 can include a plurality of elongated struts 452 coupled to the proximal portion 410a and configured to extend at least partially into the delivery device 440. In a further configuration, the struts 452 of the retrieval member 450 can be extensions of the struts 412 of the outer member 410. In further configurations, the retrieval member 450 may include additional structural elements, such as a hook or other similar elements.

Pushing the retrieval member 450 in a distal direction relative to the delivery device 440 can force the interluminal medical treatment device 400 out of the delivery device 440 and into a deployed position. Alternatively, pulling the retrieval member 450 in a proximal direction relative to the delivery device 440 can force the interluminal medical treatment device 400 into the delivery device 440 and into a collapsed position. Accordingly, the retrieval member 450 may assist in the deployment and retrieval of the interluminal medical treatment device 400.

As introduced above, the interluminal medical treatment device 400 can include an inner member 470 extending longitudinally along the length of the interluminal medical treatment device 400 and being disposed within the membranes 420a, 420b and/or outer member 410. In particular, the inner member 470 can be coupled to an inner surface of the membranes 420a, 420b and/or outer member 410. The inner member 470 may comprise any of a number of different materials such as metals, plastics, and/or similar materials, and/or combinations thereof, including any materials that may be used for the outer member 410. Furthermore, the materials used to form the inner member 470 may be flexible or rigid.

The inner member 470 may define and/or contain one or more lumens. For example, the inner member 470 may include a perfusion lumen 472, a beneficial agent lumen 474, and a guidewire lumen 476. As shown in the example configuration, the beneficial agent lumen 474 and/or guidewire lumen 476 can be disposed within the perfusion lumen 472.

The inner member 470 may include one or more perfusion ports 482 in fluid communication with the perfusion lumen 472. For example, the inner member may include one or more perfusion ports 482 proximal of the housing area 430 to allow fluid flow within the body lumen 490 that has been redirected by the membranes 420a, 420b and/or outer member 410 to enter into and travel through the perfusion lumen 472 of the inner member 470. The inner member 470 may also include one or more perfusion ports 482 distal of the housing area 430 to allow fluid flow to exit the perfusion lumen 472. As a result, the housing area 430 can be isolated and protected as fluid flow within the body lumen 490 is maintained and redirected through the inner member 470.

The inner member 470 can include one or more beneficial agent ports 484 in fluid communication with the beneficial agent lumen 474 and extending into the housing area 430. Once the interluminal medical treatment device 400 is deployed, one or more beneficial agents can be introduced into the housing area 430 via the beneficial agent lumen 474 and corresponding port(s) 484. As a result, the beneficial agent(s) can be used to treat a targeted treatment site within the body lumen 490. In a further configuration, the inner member 470 may include a plurality of beneficial agent lumens 474 with corresponding beneficial agent ports 484 extending into the housing area 430 for the delivery of a plurality of distinct beneficial agents into the housing area 430.

The beneficial agent lumen 474 may extend from a point external to a patient receiving treatment through the lumen 490 and into the housing area 430. As a result, a physician may use a syringe or other external device to introduce a beneficial agent into the housing area 430 through the beneficial agent lumen 474. In one example embodiment, the injection device 460 can be coupled to an automated device, whether internal or external to the patient, configured to automatically inject a predetermined amount of beneficial agent into the housing area 430 at predetermined time intervals.

The inner member 470 may include a guidewire lumen 476 extending longitudinally therein. A corresponding guidewire 475 may pass through the guidewire lumen 476 and be used to position the interluminal medical treatment device 400 proximate a targeted treatment site within the body lumen 490. Once the interluminal medical treatment device 400 is positioned and/or deployed, the guidewire 475 may be removed from or remain within the guidewire lumen 476.

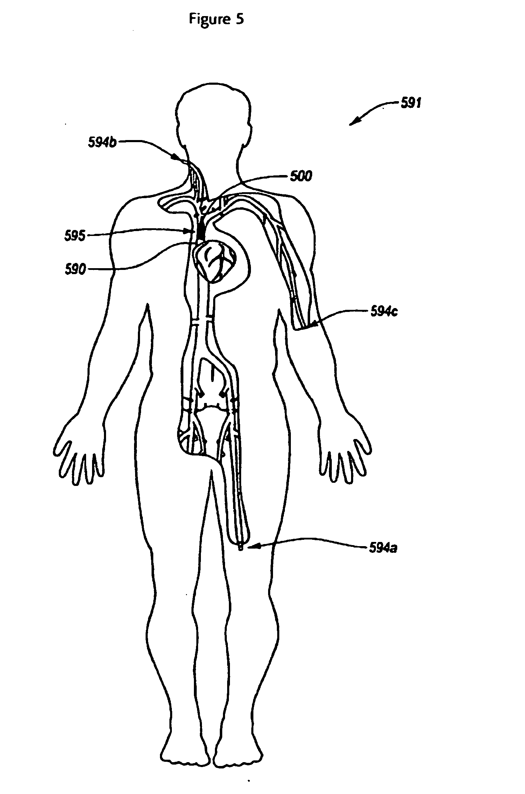

FIG. 5 illustrates an exemplary subject 591 for an interluminal medical treatment device 500. The interluminal medical treatment device 500 may be functionally similar to the interluminal medical treatment devices 100, 200, 300, and 400 previously described above and shown in FIGS. 1-4 in most respects, wherein certain features will not be described in relation to this configuration wherein those components may function in the manner as described above and are hereby incorporated into the configuration described below. Like structures and/or components are given like reference numerals. Additionally, the interluminal medical treatment device 500 may incorporate at least one component of the interluminal medical treatment devices 100, 200, 300, 400, and 600 described in connection with FIGS. 1-4 and 6, respectively.

The interluminal medical treatment device 500 may be implanted in a body lumen 590 of the subject 591. As illustrated in FIG. 5, the interluminal medical treatment device 500 may be inserted and/or retrieved through an access site 594a, 594b, 594c. In the present embodiment, the access site may include a femoral artery access site 594a, a jugular vein access site 594b, a radial vein access site 594c, femoral vein, brachial vein, brachial artery, other access sites, or combinations thereof.

For instance, the interluminal medical treatment device 500 may be inserted through the femoral artery access site 594a and retrieved through the jugular or radial vein access site 594b, 594c. In another example, the interluminal medical treatment device 500 may be inserted through the jugular vein access site 594b and retrieved through the femoral artery or radial vein access site 594a, 594c. In a further example, the interluminal medical treatment device 500 may be inserted through the radial vein access site 594c and retrieved through the femoral artery or jugular vein access site 594a, 594b.

In a yet further example, the interluminal medical treatment device 500 may be inserted and retrieved through the radial vein access site 594c. Additionally, the interluminal medical treatment device 500 may be inserted and retrieved through the jugular vein access site 594b. Further, the interluminal medical treatment device 500 may be inserted and retrieved through the femoral artery access site 594a.

The interluminal medical treatment device 500 may be deployed near a deployment site 595. In one implementation, the deployment site 595 may include a location within a coronary artery. In other implementations, other deployment sites may be used. For example, the deployment site 595 may include central and peripheral arteries and veins, vena cavas, bile ducts, esophagus, colons, trachea, large bronchi, ureters, and urethra.

Once deployed at the deployment site 595, the interluminal medical treatment device 500 may at least partially define a housing area (not shown) adjacent to the inner wall of the body lumen 590. A medical care provider can introduce a beneficial agent into the housing area 530, which may be at least partially protected from fluid flow within the body lumen 590.

In one implementation, the interluminal medical treatment device 500 may be coupled to a beneficial agent lumen (not shown). In particular, the beneficial agent lumen can be part of or coupled to an injection device (e.g., 260, FIGS. 2A-2C) or an inner member (e.g., 370, 470, FIGS. 3-4) of the interluminal medical treatment device 500. The beneficial agent lumen may extend from the deployed interluminal medical treatment device 500 to a point external to the subject 591 through one of the access sites 594a, 594b, 594c. For example, the beneficial agent lumen can extend from the deployment site 595 through the radial vein access site 594c to a point external to the subject 591. Accordingly, a physician or other medical care provider can access the beneficial agent lumen in order to introduce one or more beneficial agents into the housing area 530 through the beneficial agent lumen.

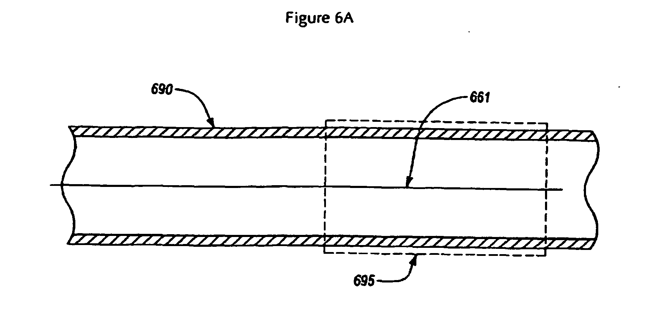

FIGS. 6A-6F illustrate various steps in the deployment of an interluminal medical treatment device 600. The interluminal medical treatment device 600 may be functionally similar to the interluminal medical treatment devices 100, 200, 300, 400, 500 previously described above and shown in FIGS. 1-5 in most respects, wherein certain features will not be described in relation to this configuration wherein those components may function in the manner as described above and are hereby incorporated into the configuration described below. Like structures and/or components are given like reference numerals. Additionally, the interluminal medical treatment device 600 may incorporate at least one component of the interluminal medical treatment devices 100, 200, 300, 400, and 500 described in connection with FIGS. 1-5, respectively.

FIG. 6A illustrates a deployment site 695 within a body lumen 690 with a guidewire 661 partially inserted therethrough. The guidewire 661 may be inserted through an access site (shown as 594a, 594b, 594c in FIG. 5) toward the deployment site 695. The guidewire 661 may be used to locate the deployment site 695. Once used to locate the deployment site 695, the guidewire 661 may either remain within the lumen 690 or may be removed. In other configurations, other methods may be used in addition to or instead of a guidewire 661. For example, an imaging device, such as a fluoroscope, x-ray, and/or other imaging device may be used to locate the deployment site 695.

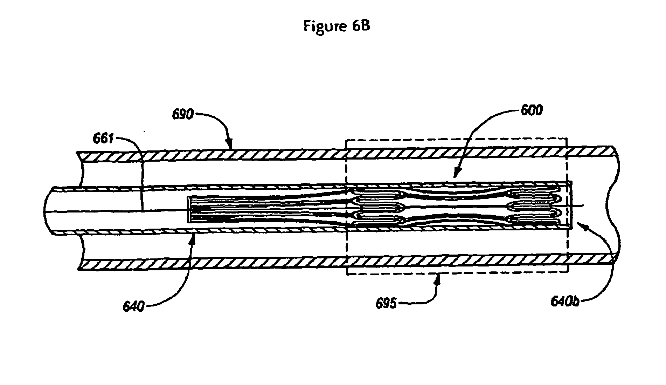

As shown in FIG. 6B, a delivery device 640 may use the guidewire 661 to guide a distal end 640b of the delivery device 640 toward the deployment site 695. An interluminal medical treatment device 600 may be disposed within the delivery device 640 in a collapsed state. While in the collapsed state, the interluminal medical treatment device 600 may be radially compressed and/or longitudinally elongated with respect to a deployed state.

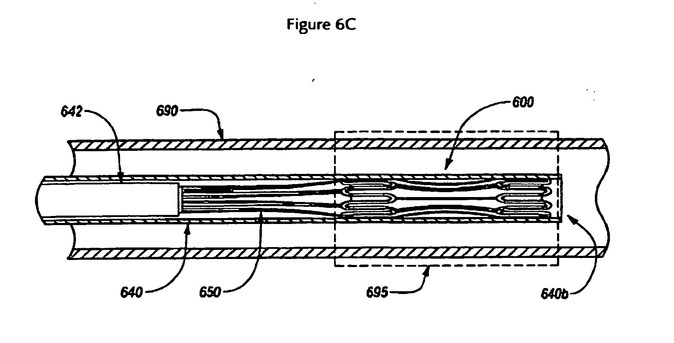

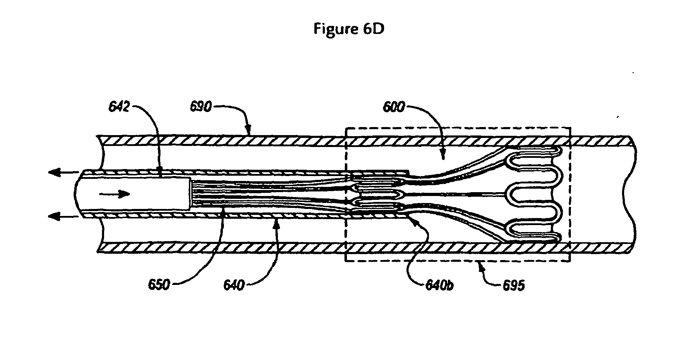

A deployment member 642 may be inserted through the delivery device 640, as shown in FIG. 6C. The deployment member 642 may be used to deploy the interluminal medical treatment device 600. In the configuration shown in FIG. 6D, the deployment member 642 may move the interluminal medical treatment device 600 toward the distal end 640b of the delivery device 640 while the delivery device 640 may remain generally stationary.

The deployment member 642 may move the interluminal medical treatment device 600 by abutting the retrieval member 650 of the interluminal medical treatment device 600. The deployment member 642 may include a receiving area (not shown), such as a convex portion configured and dimensioned to receive the retrieval member 650, to facilitate deploying the interluminal medical treatment device 600.

In one implementation, as shown in Figure 6D, the delivery device 640 may be retracted while the deployment member 642 may remain generally stationary. In a further implementation, the deployment member 642 may be advanced while the delivery device 640 may remain stationary. In other configurations, the delivery device 640 and/or the deployment member 642 may cooperate to facilitate deployment of the interluminal medical treatment device 600. For instance, the delivery device 640 may be retracted while the deployment member 642 may urge the interluminal medical treatment device 600 toward the distal end 640b of the delivery device 640.

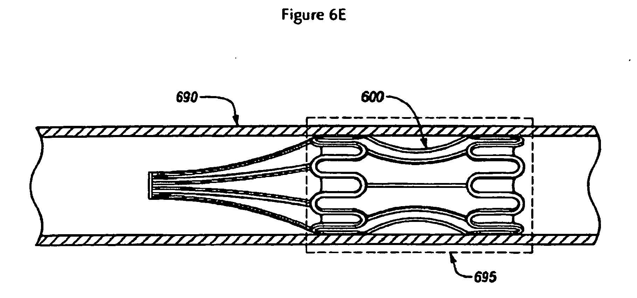

FIG. 6E illustrates a deployed interluminal medical treatment device 600 within the body lumen 690. In the deployed configuration, the implantable interluminal medical treatment device 600 may engage an inside surface of the body lumen 690. For example, the proximal portion 610a and distal portion 610b of the interluminal medical treatment device 600 may engage the inside surface of the body lumen 690. In the deployed configuration, the interluminal medical treatment device 600 may be longitudinally reduced with respect to a collapsed configuration.

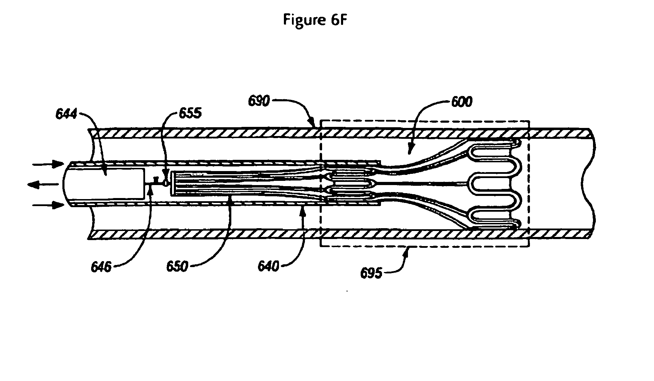

In a further implementation, as shown in FIG. 6F, the interluminal medical treatment device 600 may include a retrieval mechanism 655 operatively connected to the retrieval member 650. The interluminal medical treatment device 600 may be engaged by a retrieval apparatus 644 disposed within the delivery device 640. The retrieval apparatus 644 may also include a retrieving mechanism 646, such as a hook and/or other retaining mechanism, configured to engage the retrieval mechanism 655 of the interluminal medical treatment device 600.

Upon engaging the retrieval mechanism 655, the retrieval apparatus 644 may urge the interluminal medical treatment device 600 into the delivery device 640. For example, urging the interluminal medical treatment device 600 toward the delivery device 640 may facilitate disengaging the interluminal medical treatment device 600 from the inside surface of the lumen 690.

In one implementation, the retrieval apparatus 644 may urge the interluminal medical treatment device 600 in a proximal direction while the delivery device 640 remains stationary. In a further implementation, the delivery device 640 may be advanced in a distal direction while the retrieval apparatus 644 maintains the interluminal medical treatment device 600 stationary. In a yet further configuration, the delivery device 640 and the retrieval apparatus 644 may both move in generally opposite directions to urge the interluminal medical treatment device 600 into the delivery device 640 into a compressed state.

Once the implantable device 600 is within the delivery device 640, the delivery device 640 and implantable device 600 may be withdrawn through an access site (shown as 594a, 594b, 594c in FIG. 5).

While the present disclosure has been described, disclosed, illustrated and shown in various terms of certain embodiments or modifications, the scope of the present disclosure is not intended to be, nor should it be deemed to be, limited thereby and such other modifications or embodiments as may be suggested by the teachings herein are particularly reserved especially as they fall within the breadth and scope of the claims here appended.

## Claims

1. An intraluminal medical treatment device (100; 200; 300; 500; 600) configured to treat a targeted treatment site within a lumen (190; 290; 390; 590; 690), comprising:
an elongated generally tubular outer member (110; 210; 310) comprising an expandable proximal portion (110a; 210a; 310a), an expandable distal portion (110b; 210b; 310b), and a central portion (110c; 210c; 310c), wherein the central portion has a lesser expandability than the proximal portion and the distal portion, and wherein
the central portion includes a plurality of elongated, c-shaped and/or semicircular or v-shaped struts connecting the proximal portion and the distal portion and wherein the elongated struts of the central portion taper and/or curve radially inward from the proximal portion and distal portion to the middle of the central portion, such that the central portion has a smaller diameter than the proximal or distal portion;
at least one membrane (120; 220; 320) coupled to the outer member, wherein the outer member and the at least one membrane at least partially define a housing area (130; 230; 330; 530) extending around the central portion of the outer member and positioned longitudinally between the expanded proximal portion and the expanded distal portion of the outer member.

2. The device according to claim 1 further comprising an elongated generally tubular inner member (370) disposed within the outer member and at least one membrane, the inner member including at least one lumen (372, 374, 376).

3. The device according to any of claims 1 or 2 further comprising an injection device (260; 360) extending at least partly into the housing area;
or a beneficial agent port (384) comprised by the inner member, wherein a beneficial agent lumen (374) is being in fluid communication with the housing area by way of the beneficial agent port.

4. The device according to any of claims 1 to 3, wherein the at least one membrane is permeable to gas and/or oxygen.

5. The device according to any of claims 1 to 4, wherein the at least one membrane is comprising or is consisting of material selected from the group comprising polymers, polyurethanes, synthetic fabrics, elastomers, polyamides, silicone, PTFE, and/or ePTFE.

6. The device according to any of claims 2 to 5, wherein the at least one lumen comprised in the inner member is selected from the group comprising a perfusion lumen (372), a beneficial agent lumen (374), and/or a guidewire lumen (376).

7. The device according to claim 6, wherein the beneficial agent lumen, and/or the guidewire lumen is disposed within the perfusion lumen.

8. The device according to claim 7, wherein the guidewire lumen is joined with the perfusion lumen.

9. The device according to any of claims 2 to 8, wherein the size of the perfusion lumen may vary or be varied longitudinally.

10. The device according to any of claims 2 to 9, wherein the at least one lumen comprised in the inner member is comprising a perfusion lumen (372) and the inner member includes at least one perfusion port (382) in a proximal portion of the inner member and at least on one perfusion port in a distal portion of the inner member, the perfusion ports being in fluid communication with the perfusion lumen, and wherein the diameter of the perfusion lumen between at least one perfusion port in a proximal portion of the inner member and at least one perfusion port in a distal portion of the inner member is greater than the diameter of the remaining inner member not comprising a perfusion lumen.

11. The device according to any of claim I to 10, further comprising at least one other medical device selected from the group of stent, stent-graft, implant, balloon or filter-device; and which preferably is a stent, especially a balloon expandable or self-expanding stent, or is a balloon.

12. The device according to claim 11, wherein the at least one other medical device is disposed circumferentially outside the outer member and between the proximal and the distal portion of the outer member.

13. The device according to any of claims 11 or 12, wherein the at least one other medical device is made of a self-expanding material or shape memory material, preferably is a self-expanding stent.

14. The device according to any of claim 11 to 13, wherein the surface of the at least one other medical device is suitable to be loaded with at least one beneficial agents.

15. The device according to any of claim 1 to 13, wherein the at least one other medical device is a balloon, a balloon-expandable stent or a balloon-expandable stent graft, wherein further the inner member comprises an inflation lumen as additional lumen.

## Patentansprüche

1. Intraluminale medizinische Behandlungsvorrichtung (100; 200; 300; 500; 600) konfiguriert zum Behandeln einer gezielten Behandlungsstelle innerhalb eines Lumens (190; 290; 390; 590; 690), umfassend:
ein längliches, im Allgemeinen röhrenförmiges äußeres Element (110; 210; 310) umfassend einen ausdehnbaren proximalen Abschnitt (110a; 210a;
310a), einen ausdehnbaren distalen Abschnitt (110b; 210b; 310b) und einen zentralen Abschnitt (110c; 210c; 310c), wobei der zentrale Abschnitt eine geringere Ausdehnbarkeit als der proximale Abschnitt und der distale Abschnitt aufweist, und
wobei der zentrale Abschnitt mehrere längliche, c-förmige und/oder halbrunde oder v-förmige Streben enthält, die den proximalen Abschnitt und
den distalen Abschnitt verbinden, und wobei sich die länglichen Streben des zentralen Abschnittes vom proximalen Abschnitt und vom distalen Abschnitt nach innen zur Mitte des zentralen Abschnittes hin verjüngen und/oder radial krümmen, derart, dass der zentrale Abschnitt einen geringeren Durchmesser als der proximale oder distale Abschnitt aufweist;
mindestens eine Membran (120; 220; 320) gekoppelt an das äußere Element, wobei das äußere Element und die mindestens eine Membran mindestens teilweise einen Gehäusebereich (130; 230; 330; 530) definieren, der sich um den zentralen Abschnitt des äußeren Elementes herum erstreckt und längs zwischen dem ausgedehnten proximalen Abschnitt und
dem ausgedehnten distalen Abschnitt des äußeren Elementes positioniert ist.

2. Vorrichtung nach Anspruch 1, ferner umfassend ein längliches, im Allgemeinen röhrenförmiges inneres Element (370) angeordnet innerhalb des äußeren Elementes und mindestens einer Membran, wobei das innere Element mindestens ein Lumen (372, 374, 376) beinhaltet.

3. Vorrichtung nach einem der Ansprüche 1 oder 2, ferner umfassend eine Injektionsvorrichtung (260; 360), die sich mindestens teilweise in den Gehäusebereich erstreckt;
oder einen vorteilhaften Wirkstoffport (384) enthalten in dem inneren Element, wobei ein vorteilhaftes Wirkstofflumen (374) über den vorteilhaften Wirkstoffport in Fluidkommunikation mit dem Gehäusebereich steht.

4. Vorrichtung nach einem der Ansprüche 1 bis 3, wobei die mindestens eine Membran durchlässig für Gas und/oder Sauerstoff ist.

5. Vorrichtung nach einem der Ansprüche 1 bis 4, wobei die mindestens eine Membran Material ausgewählt aus der Gruppe umfassend Polymere, Polyurethane, synthetische Stoffe, Elastomere, Polyamide, Silikon, PTFE und/oder ePTFE umfasst oder daraus besteht.

6. Vorrichtung nach einem der Ansprüche 2 bis 5, wobei das mindestens eine Lumen enthalten in dem inneren Element ausgewählt ist aus der Gruppe umfassend ein Perfusionslumen (372), ein vorteilhaftes Wirkstofflumen (374) und/oder ein Führungsdrahtlumen (376).

7. Vorrichtung nach Anspruch 6, wobei das vorteilhafte Wirkstofflumen und/oder das Führungsdrahtlumen innerhalb des Perfusionslumens angeordnet sind.

8. Vorrichtung nach Anspruch 7, wobei das Führungsdrahtlumen mit dem Perfusionslumen verbunden ist.

9. Vorrichtung nach einem der Ansprüche 2 bis 8, wobei die Größe des Perfusionslumens variieren kann oder in Längsrichtung variiert sein kann.

10. Vorrichtung nach einem der Ansprüche 2 bis 9, wobei das mindestens eine Lumen enthalten in dem inneren Element ein Perfusionslumen (372) umfasst und das innere Element mindestens einen Perfusionsport (382) in einem proximalen Abschnitt des inneren Elementes und mindestens einen Perfusionsport in einem distalen Abschnitt des inneren Elementes enthält, wobei die Perfusionsports in Fluidkommunikation mit dem Perfusionslumen stehen, und wobei der Durchmesser des Perfusionslumens zwischen mindestens einem Perfusionsport in einem proximalen Abschnitt des inneren Elementes und mindestens einem Perfusionsport in einem distalen Abschnitt des inneren Elementes größer als der Durchmesser des übrigen inneren Elementes, das kein Perfusionslumen umfasst, ist.

11. Vorrichtung nach einem der Ansprüche 1 bis 10, ferner umfassend mindestens eine andere medizinische Vorrichtung ausgewählt aus der Gruppe von Stent, Stentgraft, Implantat, Ballon oder Filtervorrichtung; und die vorzugsweise ein Stent, insbesondere ein ballonexpandierbarer oder selbstexpandierender Stent, oder ein Ballon ist.

12. Vorrichtung nach Anspruch 11, wobei die mindestens eine andere medizinische Vorrichtung umlaufend außerhalb des äußeren Elementes und zwischen dem proximalen und dem distalen Abschnitt des äußeren Elementes angeordnet ist.

13. Vorrichtung nach einem der Ansprüche 11 oder 12, wobei die mindestens eine andere medizinische Vorrichtung aus einem selbstexpandierenden Material oder Formgedächtnismaterial hergestellt ist, und vorzugsweise ein selbstexpandierender Stent ist.

14. Vorrichtung nach einem der Ansprüche 11 bis 13, wobei die Oberfläche der mindestens einen anderen medizinischen Vorrichtung zum Beladen mit mindestens einem vorteilhaften Wirkstoff geeignet ist.

15. Vorrichtung nach einem der Ansprüche 1 bis 13, wobei die mindestens eine andere medizinische Vorrichtung ein Ballon, ein ballonexpandierbarer Stent oder ein ballonexpandierbarer Stentgraft ist, wobei ferner das innere Element ein Inflationslumen als zusätzliches Lumen umfasst.

## Revendications

1. Dispositif de traitement médical intraluminal (100 ; 200 ; 300 ; 500 ; 600) configuré pour traiter un site de traitement cible à l'intérieur d'une lumière (190 ; 290 ; 390 ; 590 ; 690), qui comprend:
un élément extérieur allongé généralement tubulaire (110 ; 210 ; 310) comprenant une partie proximale expansible (110a ; 210a ; 310a), une partie distale expansible (110b ; 210b ; 310b), et
une partie centrale (110c ; 210c ; 310c), dans lequel la partie centrale est moins expansible que la partie proximale et la partie distale, et dans lequel la partie centrale comprend une pluralité d'entretoises allongées, en forme de c et/ou semi-circulaires ou en forme de v reliant la partie proximale et la partie distale et dans lequel les entretoises allongées de la partie centrale diminuent et/ou s'incurvent radialement vers l'intérieur depuis la partie proximale et la partie distale en direction du milieu de la partie centrale, de telle manière que la partie centrale a un diamètre plus petit que la partie proximale ou la partie distale ;
au moins une membrane (120 ; 220 ; 320) couplée à l'élément extérieur, dans lequel l'élément extérieur et la ou les membranes définissent au moins partiellement une zone de logement (130 ; 230 ; 330 ; 530) s'étendant autour de la partie centrale de l'élément extérieur et positionnée longitudinalement entre la partie proximale déployée et la partie distale déployée de l'élément extérieur.

2. Dispositif selon la revendication 1, comprenant en outre un élément intérieur tubulaire généralement allongé (370) disposé à l'intérieur de l'élément extérieur et au moins une membrane, l'élément intérieur comprenant au moins une lumière (372, 374, 376).

3. Dispositif selon l'une quelconque des revendications 1 ou 2, comprenant en outre un dispositif d'injection (260 ; 360) s'étendant au moins partiellement dans la zone de logement ;
ou un orifice pour agent bénéfique (384) compris dans l'élément intérieur, dans lequel une lumière pour agent bénéfique (374) se trouve en communication fluidique avec la zone de logement au moyen de l'orifice pour agent bénéfique.

4. Dispositif selon l'une quelconque des revendications 1 à 3, dans lequel la ou les membranes sont perméables au gaz et/ou à l'oxygène.

5. Dispositif selon l'une quelconque des revendications 1 à 4, dans lequel la ou les membranes comprennent ou sont constituées d'un matériau choisi dans le groupe comprenant des polymères, des polyuréthanes, des tissus synthétiques, des élastomères, des polyamides, du silicone, du PTFE, et/ou de l'ePTFE.

6. Dispositif selon l'une quelconque des revendications 2 à 5, dans lequel la ou les lumières comprises dans l'élément intérieur sont choisies dans le groupe comprenant une lumière de perfusion (372), une lumière pour agent bénéfique (374), et/ou une lumière pour fil-guide (376).

7. Dispositif selon la revendication 6, dans lequel la lumière pour agent bénéfique, et/ou la lumière pour fil-guide sont disposées à l'intérieur de la lumière de perfusion.

8. Dispositif selon la revendication 7, dans lequel la lumière pour fil-guide est jointe à la lumière de perfusion.

9. Dispositif selon l'une quelconque des revendications 2 à 8, dans lequel la taille de la lumière de perfusion peut varier ou être variable longitudinalement.

10. Dispositif selon l'une quelconque des revendications 2 à 9, dans lequel la ou les lumières comprises dans l'élément intérieur comprennent une lumière de perfusion (372) et l'élément intérieur comprend au moins un orifice de perfusion (382) dans une partie proximale de l'élément intérieur, et au moins un orifice de perfusion dans une partie distale de l'élément intérieur, les orifices de perfusion étant en communication fluidique avec la lumière de perfusion, et dans lequel le diamètre de la lumière de perfusion entre le ou les orifices de perfusion dans une partie proximale de l'élément intérieur et au moins un orifice de perfusion dans une partie distale de l'élément intérieur est supérieur au diamètre du reste de l'élément intérieur ne comprenant pas de lumière de perfusion.

11. Dispositif selon l'une quelconque des revendications 1 à 10, comprenant en outre au moins un autre dispositif médical choisi dans le groupe comprenant un stent, une endoprothèse, un implant, un ballonnet ou un dispositif de filtration ; ledit autre dispositif médical étant de préférence un stent, notamment un stent expansible par ballonnet ou auto-expansible, ou étant un ballonnet.

12. Dispositif selon la revendication 11, dans lequel le ou les dispositifs médicaux sont disposés sur la circonférence à l'extérieur de l'élément extérieur et entre la partie proximale et la partie distale de l'élément extérieur.

13. Dispositif selon l'une quelconque des revendications 11 ou 12, dans lequel le ou les autres dispositifs médicaux sont constitués d'un matériau auto-expansible ou d'un matériau à mémoire de forme, et sont de préférence un stent auto-expansible.

14. Dispositif selon l'une quelconque des revendications 11 à 13, dans lequel la surface du ou des autres dispositifs médicaux est adaptée à être chargée d'un ou de plusieurs agents bénéfiques.

15. Dispositif selon l'une quelconque des revendications 1 à 13, dans lequel le ou les dispositifs médicaux sont un ballonnet, un stent expansible par ballonnet ou une endoprothèse expansible par ballonnet, dans lequel l'élément intérieur comprend en outre une lumière de gonflement en tant que lumière supplémentaire.
